(19) **Europäisches Patentamt / European Patent Office / Office européen des brevets**

(11) **EP 3 283 628 B1**

(12) # EUROPEAN PATENT SPECIFICATION

(45) Date of publication and mention
of the grant of the patent:
**12.02.2020 Bulletin 2020/07**

(51) Int Cl.:
*C12N 15/10* [(2006.01)]  *C12N 15/63* [(2006.01)]
*C12N 15/67* [(2006.01)]  *C12Q 1/68* [(2018.01)]

(21) Application number: **16716591.9**

(22) Date of filing: **15.04.2016**

(86) International application number:
**PCT/EP2016/058383**

(87) International publication number:
**WO 2016/166310 (20.10.2016 Gazette 2016/42)**

(54) **RIBOSWITCH INDUCIBLE GENE EXPRESSION**

RIBOSWITCH-INDUZIERBARE GENEXPRESSION

EXPRESSION GÉNIQUE INDUCTIBLE PAR RIBORÉGULATEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.04.2015 GB 201506507**

(43) Date of publication of application:
**21.02.2018 Bulletin 2018/08**

(73) Proprietor: **Wageningen Universiteit
6708 PB Wageningen (NL)**

(72) Inventors:
• **CREUTZBURG, Sjoerd Constantijn Arnoud
6708 PZ Wageningen (NL)**
• **VAN DER OOST, John
6871 HZ Renkum (NL)**

(74) Representative: **HGF Limited
8th Floor
140 London Wall
London EC2Y 5DN (GB)**

(56) References cited:
WO-A1-2012/021554      WO-A2-01/64956
WO-A2-2008/116220      WO-A2-2012/153142

• **THOMPSON K M ET AL: "GROUP I APTAZYMES AS GENETIC REGULATORY SWITCHES", BMC BIOTECHNOLOGY, BIOMED CENTRAL LTD. LONDON, GB, vol. 2, 4 December 2002 (2002-12-04), pages 1-12, XP008062371, ISSN: 1472-6750, DOI: 10.1186/1472-6750-2-21 cited in the application**

• **CHEAH M T ET AL: "Control of alternative RNA splicing and gene expression by eukaryotic riboswitches", NATURE, NATURE PUBLISHING GROUP, UNITED KINGDOM, vol. 447, no. 7143, 24 May 2007 (2007-05-24), pages 497-500,1, XP002584633, ISSN: 0028-0836, DOI: 10.1038/NATURE05769 [retrieved on 2007-04-29]**

• **VAN ROSSUM TEUNKE ET AL: "Reporter-based screening and selection of enzymes.", THE FEBS JOURNAL JUL 2013, vol. 280, no. 13, July 2013 (2013-07), pages 2979-2996, XP002759900, ISSN: 1742-4658 cited in the application**

• **GROHER FLORIAN ET AL: "Synthetic riboswitches - A tool comes of age", BIOCHIMICA ET BIOPHYSICA ACTA-GENE REGULATORY MECHANISMS, vol. 1839, no. 10, Sp. Iss. SI, October 2014 (2014-10), pages 964-973, XP002759901, cited in the application**

• **CHRISTIAN BERENS ET AL: "RNA aptamers as genetic control devices: The potential of riboswitches as synthetic elements for regulating gene expression", BIOTECHNOLOGY JOURNAL, vol. 10, no. 2, 1 February 2015 (2015-02-01), pages 246-257, XP055279945, DE ISSN: 1860-6768, DOI: 10.1002/biot.201300498**

• **WELZ R ET AL: "Ligand binding and gene control characteristics of tandem riboswitches in Bacillus anthracis", RNA, COLD SPRING HARBOR LABORATORY PRESS, US, vol. 13, no. 4, 16 February 2007 (2007-02-16), pages 573-582, XP002615439, ISSN: 1355-8382, DOI: 10.1261/RNA.407707 [retrieved on 2007-02-16]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- DONG-SUK KIM ET AL: "An artificial riboswitch for controlling pre-mRNA splicing", RNA, COLD SPRING HARBOR LABORATORY PRESS, US, vol. 11, no. 11, 1 November 2005 (2005-11-01), pages 1667-1677, XP008126231, ISSN: 1355-8382, DOI: 10.1261/RNA.2162205
- DESAI S K ET AL: "Genetic screens and selections for small molecules based on a synthetic riboswitch that activates protein translation", JOURNAL OF THE AMERICAN CHEMICAL SOCIETY, AMERICAN CHEMICAL SOCIETY, US, vol. 126, 1 January 2004 (2004-01-01), pages 13,247-13,254, XP002982412, ISSN: 0002-7863, DOI: 10.1021/JA048634J cited in the application
- CHEN ANDY G Y ET AL: "Mechanism for gene control by a natural allosteric group I ribozyme.", RNA (NEW YORK, N.Y.) NOV 2011, vol. 17, no. 11, November 2011 (2011-11), pages 1967-1972, XP002759961, ISSN: 1469-9001
- E. R. LEE ET AL: "An Allosteric Self-Splicing Ribozyme Triggered by a Bacterial Second Messenger", SCIENCE, vol. 329, no. 5993, 13 August 2010 (2010-08-13), pages 845-848, XP55010727, ISSN: 0036-8075, DOI: 10.1126/science.1190713

## Description

### Field of the Invention

The invention relates to the fields of cell biology, molecular genetics and genetic engineering. More particularly, the invention relates to the art of inducer-specific gene expression including materials, methods, systems and kits for performance of inducible gene expression.

### Background to the Invention

Gene expression may be regulated by modulating the rate of transcription of DNA to RNA, (usually mRNA), or translation of mRNA into a polypeptide. Often, genes have a promoter which controls expression of a gene operably linked to that promoter region. Such promoters may be inducible in their activity by an inducer molecule, allowing for transcription of these genes to be turned on, or off, in response to the presence of inducer molecules. Recently, RNA-based gene control elements called riboswitches have attracted attention. Man-made riboswitches have been made and used.

Riboswitches are mRNA-based regulatory elements which allow for a ligand-dependent control of gene expression. A riboswitch comprises an aptamer which binds the inducer molecule (ligand). This ligand binding results in a structural change in the mRNA riboswitch which in turn may increase or decrease expression of the corresponding gene. Hence, riboswitches regulate gene expression at the translational level. For example there is the theophylline-responsive ON riboswitch for the csrA (carbon storage regulator) gene of *Escherichia coli.* This permits some control of cellular auto-aggregation and motility of the resulting *E. coli* switch-csrA mutant organism.

A number of small-molecule inducible expression systems have been developed in bacteria. These include lactose (lac), tetracycline (tet) and arabinose (ara) operons. These have been used for recombinant protein production, e.g. biopharmaceuticals and industrial biocatalysts.

Inducible expression systems are used in synthetic biology. For example, to control genetic circuits acting as sensors, as switches or as oscillators. The expression of novel metabolic pathways can be placed under control of inducible promoters, e.g. for fine chemicals, natural products - including drug precursors and fatty acids - or for biofuels. There is even the development of organisms induced by specific organic pollutants for use in bioremediation.

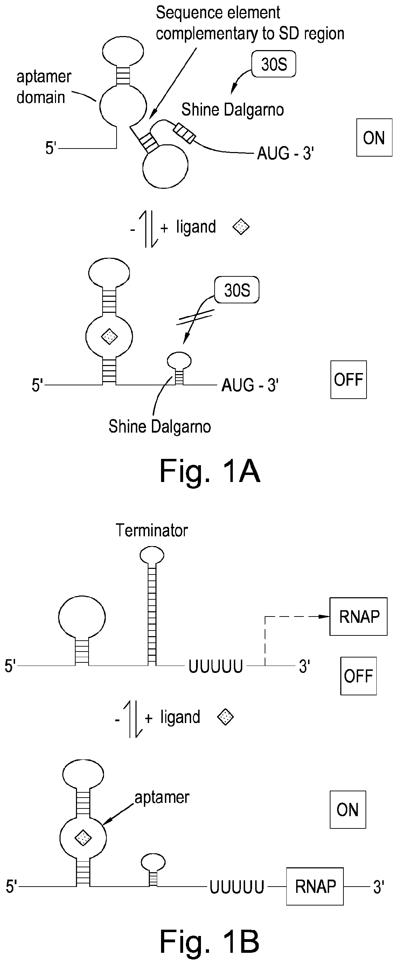

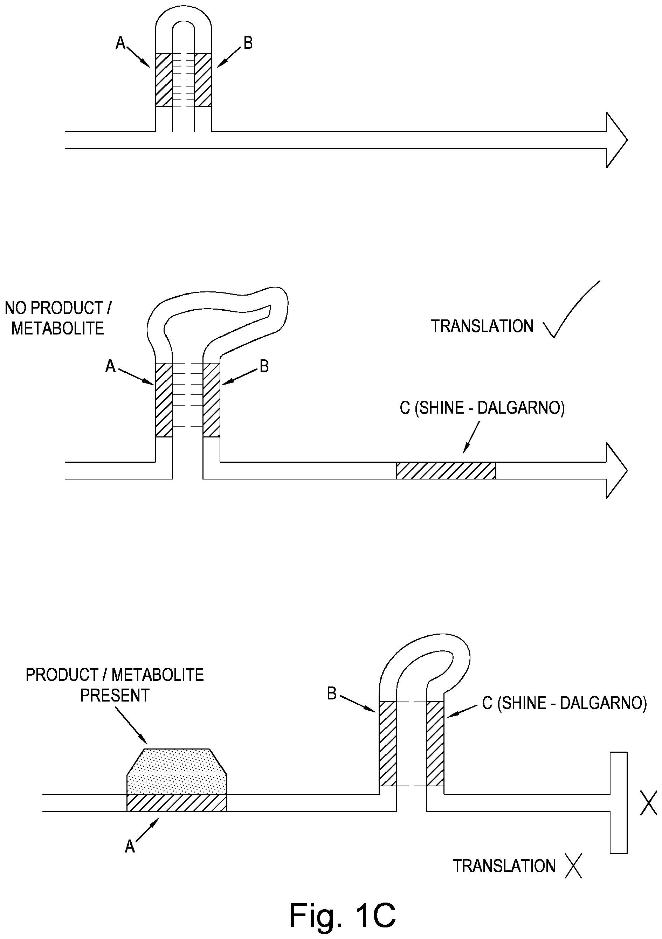

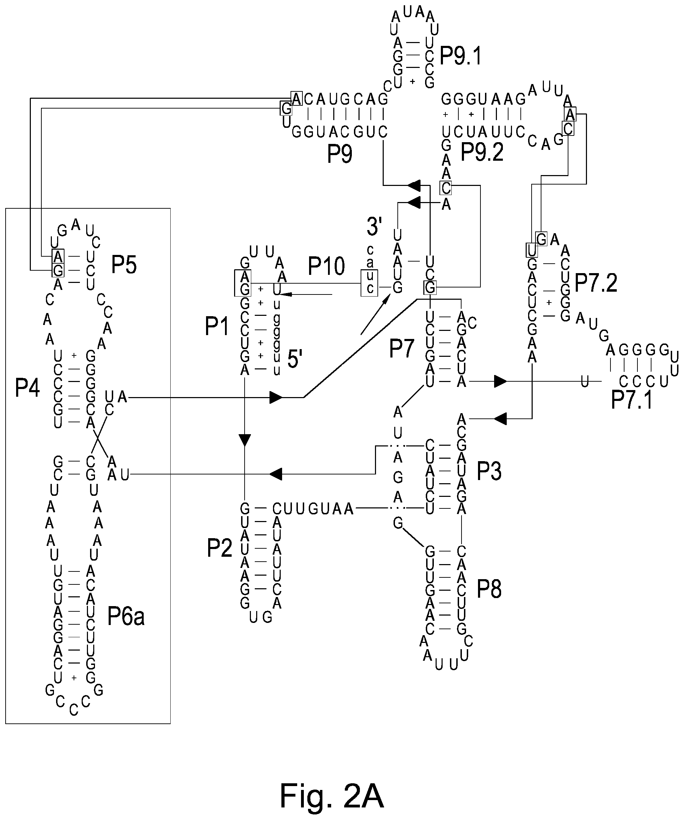

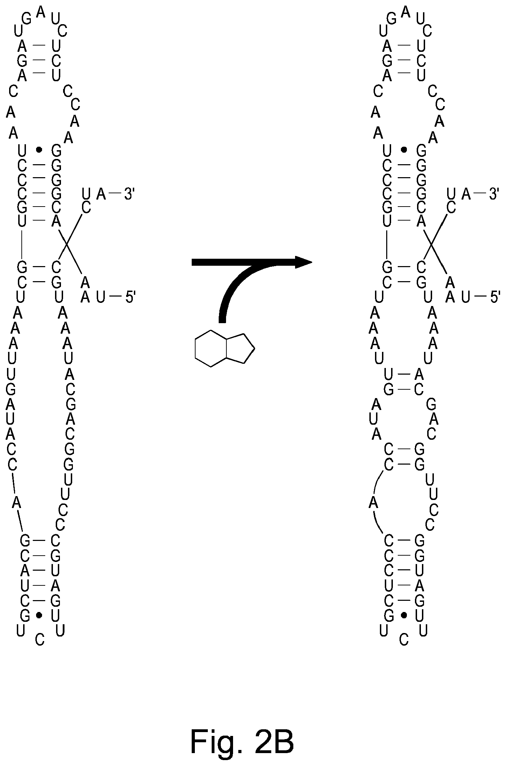

A review of current knowledge of engineered riboswitches and their application in gene expression is provided by Groher F. & Suess B. (2014) "Synthetic riboswitches - A tool comes of age." Biochimica et Biophysica Acta 1839: 964 - 973. Figure 1 of Groher & Suess illustrates and briefly describes common mechanisms of engineered riboswitches in bacteria. Figure 2 illustrates and briefly describes common mechanism in eukaryotes.

Natural riboswitches are typically located in the 5'-untranslated region (UTR) of a mRNA, controlling the translation of the downstream coding sequence. Inspired by these naturally occurring cases, several synthetic riboswitches have been developed which harness the ability of riboswitches to regulate gene expression in response to exogenously applied stimuli. Again, they reside in the 5' UTR of a reporter gene, they are induced by ligand-dependent structural rearrangements and they block translation of the reporter transcript either by masking the Shine-Dalgarno sequence or by nucleolytic cleavage by the riboswitch/ribozyme (Suess et al., 2004 Nucleic Acids Res 32:1610-1614; Ogawa and Maeda, 2007 Bioorg Med Chem Lett 17:3156-3160; Topp and Gallivan 2008 RNA 14:2498-2503; Mandal and Breaker 2004 Nat Rev Mol Cell Biol 5:451-463).

Building on the results of previous studies showing that regions of secondary structure in mRNA 5'-UTRs could cause substantial reductions in expression in prokaryotic and eukaryotic cells (Paraskeva et al. 1998 PNAS 95:951-956; Stripecke et al. 1994 Mol. Cell. Biol. 14:5898-5909; De Smit and Van Duin 1990 PNAS 87:7668-7672) ligand-inducible gene expression was accomplished in yeast by introduction of a small-molecule binding RNA into the 5'-UTR of a gene (Werstuck and Green, 1998 Science 282:296-298). This concept was extended to a variety of organisms by generating synthetic riboswitches which were responsive to theophylline (Desai and Gallivan 2004 J. Am. Chem. Soc. 126:13247-13254; Suess et al. 2004 Nucleic Acids Res. 32:1610-1614; Thompson et al. 2002 BMC Biotechnol. 2: 21), tetracyclines (Suess et al. 2003 Nucleic Acids Res. 31 :1853-1858) or dyes (Werstuck and Green, 1998 Science 282:296-298).

So far these switches have been developed with a view to remotely improving the control of heterologous gene expression in host cells and therefore the focus of this research has largely been centered on the development of high-throughput screens or selections to isolate synthetic riboswitches that respond to a variety of exogenously applied ligands (Thompson et al. 2002 BMC Biotechnol. 2002 2: 21; Ogawa and Maeda, 2007 Bioorg. Med. Chem. Lett. 17: 3156-3160).

Common gene expression systems continue to suffer drawbacks. One is all-or-nothing expression spread within a population of cells, some are fully induced, others are not. Some promoter based systems are "leaky" meaning that they have high basal (i.e. uninduced) levels of expression. This can present particular difficulties when toxic genes need to be expressed in a controlled way.

The lac and ara systems for example, exhibit cross-talk which makes them difficult to use in situations of simultaneous and differential expression of multiple genes using distinct inducers.

In the tet system, where the inducer is tetracycline or an analog, inhibition of cell growth is often a problem.

Vitamin B12, is controlled by a riboswitch that senses the intracellular concentration of vitamin B12 (see Mandal and Breaker (2004) Nat Rev Mol Cell Biol 5:451 -463).

WO 2008/116220 A2 (YALE UNIVERSITY) discloses a regulatable gene expression construct comprising a nucleic acid molecule encoding an RNA comprising a riboswitch operably linked to a coding region, wherein the riboswitch regulates splicing of the RNA, wherein the riboswitch and coding region are heterologous. The riboswitch can regulate alternative spicing of the RNA. The riboswitch can comprise an aptamer domain and an expression platform domain, wherein the aptamer domain and the expression platform domain are heterologous. The RNA can further comprise an intron, wherein the expression platform domain comprises an alternative splice junction in the intron. The alternative splice junction can be active when the riboswitch is activated by a trigger molecule, such as thiamine pyrophosphate (TPP).

Cheah M. T., et al. (2007) "Control of alternative RNA splicing and gene expression by eukaryotic riboswitches", Nature vol. 447, no. 7143, pages 497-500 describes an examination of three TPP riboswitches in the filamentous fungus Neurospora crassa; and reports that one activates and two repress gene expression by controlling mRNA splicing. A detailed mechanism involving riboswitch-mediated base-pairing changes and alternative splicing control is described for precursor *NMTI* mRNAs, which code for a protein involved in TPP metabolism. The research described demonstrates that eukaryotic cells employ metabolite-binding RNAs to regulate RNA splicing events.

WO 2012/153142 A2 (UNIVERSITY OF MANCHESTER) discloses a system comprising a genetic construct a riboswitch operably linked to a regulatory sequence, and a second genetic construct a coding sequence whose expression is capable of being regulated by a gene product of the first construct. Also provided is a genetic construct comprising one or more riboswitches for regulation of gene expression, wherein preferably a spacer sequence is provided downstream of the riboswitch to enhance expression of a coding sequence which is operably linked to a riboswitch. Ligands, kits, methods, host cells and expression systems are also provided.

The inventors configured an intronic, self-splicing riboswitch for inducible gene expression by introducing an appropriate aptamer, and then used this in an inducible gene expression system, whereby exposure of the cell to the inducer triggers self-splicing of the intron sequence to restore the reading frame of the reporter gene and as such to drive expression of the gene product.

## Summary of the Invention

The method of the invention may use a cell provided by the step of transforming a host cell with the polynucleotide expression constructs.

Accordingly, the present invention provides a method of inducing production of a desired RNA molecule or a desired protein or polypeptide in a cell comprising:

(i) providing a cell comprising a first and a second polynucleotide expression construct, the first construct comprising a polynucleotide sequence to be transcribed, and wherein the polynucleotide sequence is interrupted by at least two introns which are self-splicing introns and whose splicing activity is under the control of an aptamer, and wherein the aptamer has binding affinity for an inducer, and the second expression construct comprises a polynucleotide sequence whose expression is under the control of a promoter which is controlled or regulated by the RNA, protein or polypeptide product of the first expression construct, and the RNA product of the second expression construct is the desired RNA product molecule or the expressed protein or polypeptide expression product of the second expression construct is the desired protein or polypeptide;

(ii) exposing the transformed cell to the inducer to activate self-splicing activity of the introns and thereby to produce the desired RNA molecule or expression of the desired protein or polypeptide.

Advantageously, the inventors provide an extremely tight (substantially leakage free), inducer-specific expression method and system, in which, for example, a T7 RNA Polymerase gene is interrupted (frame-shifted) by two or more riboswitches; the frameshift is repaired upon self-splicing of the riboswitch(es) in the presence of the inducing ligand; this system may be used together with an induction of transcription, e.g. by IPTG or rhamnose
In any aspect of the invention (including methods, systems, transformed cells) as set forth herein, the presence of two expression constructs provides a "two-component" inducible expression system.

In certain embodiments of the methods of the invention, the cell may be transformed with the first and second constructs separately. In other embodiments, the cell may be transformed with first and second constructs substantially simultaneously. In yet other embodiments, the cell may be transformed with first and second constructs sequentially.

In other embodiments of the method of the invention, the second expression construct may comprise a polynucleotide sequence whose expression is under the control of a promoter which is itself controlled or regulated by the RNA product of the first expression construct, and the transcribed RNA of the second expression construct is the desired RNA.

In further embodiments of the methods of the invention, the second expression construct may comprise a polynucleotide sequence whose expression is under the control of a promoter which is controlled or regulated by the expressed RNA, protein or polypeptide of the first expression product, and expressed protein or polypeptide of the second expression construct is the desired protein or polypeptide.

In various methods of the invention, the RNA may be selected from one of a microRNA (miRNA), a small interfering RNA (siRNA), an antisense RNA, a tRNA or a ribozyme.

In any of the methods of the invention, the host cell may be a prokaryotic cell, or a eukaryotic cell.

In methods of the invention, the at least two self-splicing introns each comprise an aptamer and wherein the aptamer is the same.

In two-component inducible expression methods of the invention the polynucleotide sequence of the first expression construct may encode Phage T7 DNA dependent RNA polymerase, in which case the second expression construct may comprise the promoter PT7.

The inducer is usually a ligand. In preferred aspects of the methods of the invention, the aptamers bind theophylline which acts as the inducer ligand. Other suitable aptamers may include those which bind to tetracycline, neomycin or malachite green.

In other preferred aspects of the methods of the invention, the self-splicing intron is the T4 *td* gene self-splicing intron.

The invention also provides a cell for inducer molecule-controlled expression of a gene product, the cell comprising first and second polynucleotide expression constructs, wherein first the construct comprises a polynucleotide sequence which is interrupted by at least two introns which are self-splicing introns and whose splicing activity is under the control of an aptamer, and wherein the aptamer has binding affinity for the inducer, and the second expression construct comprises a polynucleotide sequence whose expression is under the control of a promoter whose activity is regulated by the expression product of the first expression construct.

In certain aspects, the expressed gene product may be an RNA molecule; optionally one of a microRNA (miRNA), a small interfering RNA (siRNA), an antisense RNA, a tRNA or a ribozyme.

In other aspects, the expressed gene product may be a protein or polypeptide.

The expressed product of the second expression construct may be an RNA molecule; optionally one of a microRNA (miRNA), a small interfering RNA (siRNA), an antisense RNA, a tRNA or a ribozyme. The expressed product of the second expression construct may alternatively be a protein or polypeptide.

In cells of the invention, the two or more self-splicing introns preferably contain the same aptamer, each having the same binding affinity for the inducer.

In preferred aspects, the or each self-splicing intron is the T4 *td* gene self-splicing intron.

The invention further provides a kit for preparing an host cell for inducible host cell expression of a gene product, comprising:

(i) a first polynucleotide expression construct, the first construct comprising a polynucleotide sequence to be transcribed, and wherein the polynucleotide sequence is interrupted by at least two introns which are self-splicing introns and whose splicing activity is under the control of an aptamer, and wherein the aptamer has binding affinity for an inducer;

(ii) a second polynucleotide expression construct, the second construct for receiving a polynucleotide sequence which encodes an RNA or protein or polypeptide to be expressed, and wherein the polynucleotide to be expressed is under the control or regulation of a promoter which is inducible by the transcribed polynucleotide.

Also, the invention provides a kit for inducible expression of a gene product, comprising:

(i) a host cell comprising a polynucleotide expression construct, wherein the construct comprises a polynucleotide sequence which is interrupted by at least two introns which are self-splicing introns and whose splicing activity is under the control of an aptamer, and wherein the aptamer has binding affinity for the inducer;

(ii) a second polynucleotide expression construct, the second construct for receiving a polynucleotide sequence which encodes an RNA or protein or polypeptide to be expressed, and wherein the polynucleotide to be expressed is under the control of a promoter which itself is controlled or regulated by the transcribed polynucleotide; optionally

(iii) a set of instructions for (a) inserting a polynucleotide sequence to be expressed into the second polynucleotide and/or (b) transforming the host cell with the second polynucleotide expression construct.

In certain embodiments of kits of the invention, the self-splicing intron is preferably the T4 *td* gene self-splicing intron.

Any of the kits of the invention may further comprise a container containing an inducer; optionally wherein the inducer ligand is selected from one of theophylline, tetracycline, neomycin or malachite green.

The utility of the present invention resides in the broad applicability of the methods of inducible gene expression, the polynucleotide expression vectors, transformed cells and kits; whereby any desired nucleic acid sequence can be tightly expressed under the control of an inducer molecule, in any commonly used host cell, for example prokaryotic cells, fungal cells, plant cells or animal cells.

The inducible expression methods and systems and materials of the invention are applicable for the controlled expression of any protein or polypeptide of interest; preferably tight on/off control substantially without leakage, i.e. expression in the absence of inducer ligand. Proteins of interest may typically include polypeptide macromolecules comprising 20 or more contiguous amino acid residues and may include, but are not limited to enzymes, structural proteins, binding proteins and/or surface-active proteins.

## Primary inducible expression constructs

As described above, the methods, systems, cells and kits of the invention employ a primary inducible expression construct. The primary construct comprises a polynucleotide sequence encoding an RNA molecule, a protein or a polypeptide, and wherein the polynucleotide sequence encoding these is interrupted by at least two introns, which are self-splicing introns and whose splicing activity is under the control of an aptamer, and wherein the aptamer has binding affinity for an inducer. An advantage of such constructs is that their transcription of the desired nucleic acid sequence (which contains the self-splicing introns) is only switched on in the presence of an inducer which binds to the aptamers of the self-splicing introns. This results in self-splicing activity and the generation of a relevant RNA transcript.

A further advantage arising out of the at least two self-splicing introns with aptamers for specific binding of an inducer is that there is substantially no background level translation of the desired RNA transcript, and in circumstances where the RNA transcript is translated by the cellular machinery, then substantially no background level expression of a desired protein or polypeptide. In other words, the primary inducible expression construct element of any aspect of the invention provides a tight on switch, not susceptible to background transcription or background expression of the desired protein or polypeptide beforehand. Similarly, in the absence of inducer following a period of induction, there is a tight off switch resulting in a rapid and substantial cessation of transcription or expression activity.

The expression product of the primary inducible expression construct is an intermediate in the overall system for expressing a desired gene product; in the sense that the intermediate expression product acts on a promoter or regulatory element of a second expression construct which itself transcribes/expresses the desired RNA or protein/polypeptide gene product. The invention advantageously provides methods, systems and cells which are a two-part inducible expression system with improved substantial absence of background expression levels whilst continuing to provide a tight on/off switch controlled by the presence of suitable concentration of inducer.

## Host Cells

Advantageously, the present invention is of broad applicability and host cells of the present invention may be derived from any genetically tractable organism which can be cultured. Therefore, in particular, commonly used host cell may be selected for use in accordance with the present invention including prokaryotic or eukaryotic cells which are genetically accessible and which can be cultured. The approaches defined herein for the selection of cells which express a protein of interest may be applied to those cells which are able to serve as a host for production of the protein of interest (POI)). It may therefore be applied to commonly used host cells, for example prokaryotic cells, fungal cells, plant cells and animal cells commonly used for recombinant heterologous protein expression.

Appropriate host cells may be prokaryotic or eukaryotic. Preferably, host cells will be selected from a prokaryotic cell, a fungal cell, a plant cell, a protist cell or an animal cell. Preferred host cells for use in accordance with the present invention are commonly derived from species which typically exhibit high growth rates, are easily cultured and/or transformed, display short generation times, species which have established genetic resources associated with them or species which have been selected, modified or synthesized for optimal expression of heterologous proteins under specific conditions. In preferred embodiments of the invention where the protein of interest is eventually to be used in specific industrial, agricultural, chemical or therapeutic contexts, an appropriate host cell may be selected based on the desired specific conditions or cellular context in which the protein of interest is to be deployed. Preferably the host cell will be a prokaryotic cell. In preferred embodiments the host cell is a bacterial cell. Preferably the host cell is an *Escherichia coli* (*E. coli*) cell.

## Expression Vectors

The primary and any second inducible expression construct can vary according to the recipient host cell and suitably may incorporate regulatory elements which allow expression in the host cell of interest and preferably facilitate high-levels of expression upon induction. Such regulatory sequences may be capable of influencing transcription or translation of a gene or gene product, for example in terms of initiation, accuracy, rate, stability, downstream processing and mobility.

Such elements may include, for example, strong and/or constitutive promoters, 5' and 3' UTR's, transcriptional and/or translational enhancers, transcription factor or protein binding sequences, start sites and termination sequences, ribosome binding sites, recombination sites, polyadenylation sequences, sense or antisense sequences, sequences ensuring correct initiation of transcription and optionally poly-A signals ensuring termination of transcription and transcript stabilisation in the host cell. The regulatory sequences may be plant-, animal-. bacteria-, fungal- or virus-derived, and preferably may be derived from the same organism as the host cell. Clearly, appropriate regulatory elements will vary according to the host cell of interest. For example, regulatory elements which facilitate high-level expression in prokaryotic host cells such as in *E. coli* may include the pLac, T7, P(Bla), P(Cat), P(Kat), trp or tac promoters. Regulatory elements which facilitate high-level expression in eukaryotic host cells might include the AOX1 or GAL1 promoter in yeast or the CMV- or SV40-promoters, CMV-enhancer, SV40-enhancer, *Herpes simplex virus* VIP16 transcriptional activator or inclusion of a globin intron in animal cells. In plants, constitutive high-level expression may be obtained using, for example, the *Zea mays* ubiquitin 1 promoter or 35S and 19S promoters of cauliflower mosaic virus (CaMV).

Suitable regulatory elements may be constitutive, whereby they direct expression under most environmental conditions or developmental stages or developmental stage specific.

In the secondary expression constructs or vectors of the invention, there is preferably an inducible promoter. The inducer is the transcription product (an RNA molecule) or an expression product (protein or polypeptide) of the primary inducible expression construct. What this provides is a two-component inducible expression system.

**Transformation of host cell with the expression constructs**

Expression constructs (primary or secondary) may be located in plasmids (expression vectors) which are used to transform the host cell. Methods of transformation may include but are not limited to; heat shock, electroporation, particle bombardment, chemical induction, microinjection and viral transformation.

A host cell may first be transformed with a primary inducible expression construct, and then followed by transforming the cell with the second expression construct. Alternatively the host cell is transformed substantially simultaneously with the primary construct and the secondary construct.

Throughout, the term "polynucleotide" as used herein refers to a deoxyribonucleotide or ribonucleotide polymer in single- or double-stranded form, or sense or anti-sense, and encompasses analogues of naturally occurring nucleotides that hybridize to nucleic acids in a manner similar to naturally occurring nucleotides. Such polynucleotides may be derived from any organism, including the host organism, or may be synthesised *de novo.* The provision of a polynucleotide may comprise synthesis of a polynucleotide. This may be for example by modification of a pre-existing sequence, e.g. by site-directed mutagenesis or possibly by *de novo* synthesis.

In all embodiments of the invention, polynucleotide sequences encoding the RNA, protein or polypeptide of interest may be prepared by any suitable method known to those of ordinary skill in the art, including but not limited to, for example, direct chemical synthesis or cloning for introduction into a desired host cell. Alternatively, the starting polynucleotide sequence may be provided and subsequently modified ex *vivo* or alternatively *in vivo* for example by site directed mutagenesis or gene editing techniques.

**Self-splicing Introns**

Advantageously, methods, systems and kits of the present invention which employ just a primary expression construct or vector are based on having a gene of interest to be transcribed/expressed in a transformed host cell, and which is interrupted by two or more riboswitches with adjustable ribonuclease (RNase) activity and/or adjustable RNA ligase activity; i.e. "self-splicing introns". When inducer is applied to the host cell it binds to each of the aptamers thereby resulting in self-splicing intron activity.

When self-splicing of the mRNA transcript occurs, this restores the open reading frame that results in functional expression of a desired protein or polypeptide.

Therefore, in the presence of an inducer, splicing of each intron will be induced, restoring the reading frame of the polynucleotide of interest, resulting in faithful translation and therefore expression of the desired protein/polypeptide.

**Aptamers**

Aptamers as used in the present invention are polynucleotide sequences which have a high binding affinity for the inducer used. The aptamers may be DNA, cDNA, RNA, preferably RNA. Suitable aptamers of the present invention are preferably 20-30nt in length; optionally they are 20nt, 21nt, 22nt, 23nt, 24nt, 25nt, 26nt, 27nt, 28nt, 29nt or 30nt in length.

Advantageously, the present invention makes use of tandem self-splicing riboswitches; stretches of RNA that can adopt different conformational states, depending on the presence or absence of an inducing molecule which binds to an aptamer portion of each riboswitch. In their natural function, riboswitches are not usually required to completely switch off expression of their genes and therefore the control of gene expression exerted by natural riboswitches is known to be incomplete. However, background levels of cell survival, growth and/or marking of the cells (where visual detection of a reporter is used) due to incomplete riboswitch control of expression (for example in the absence of the binding product), negatively influences the efficiency of inducible expression systems.

For the purpose of developing an accurate screening or selection system, a tightly controlled on/off switch is desirable. Advantageously, the present invention employs synthetic riboswitches with improved stringency, whereby two, optionally more than two, i.e. multiple copies of the riboswitches in sequential arrangement are used.

The self-splicing riboswitches whose splicing is under the control of an aptamer, have the effect in use of reducing background levels of expression of genes into which they have been introduced. Reductions in background level expression compared to the UTR located riboswitch constructs or existing known two-component expression systems is preferably at least about 50% (i.e. only about 50% of the background level expression of the existing constructs or systems), more preferably at least about 75%, more preferably at least about 80% reduction.

When two self-splicing introns are present as the riboswitches under aptamer control, then when compared to equivalent constructs and systems where there is just one riboswitch under the aptamer control, then the background level of growth of cells is reduced, by at least 75%, at least 80%, at least 85%, at least 90%, at least 95%. Reductions of at least about 99.5%, at least about 99.9% or even 100% (i.e. no background level expression) are optionally preferred.

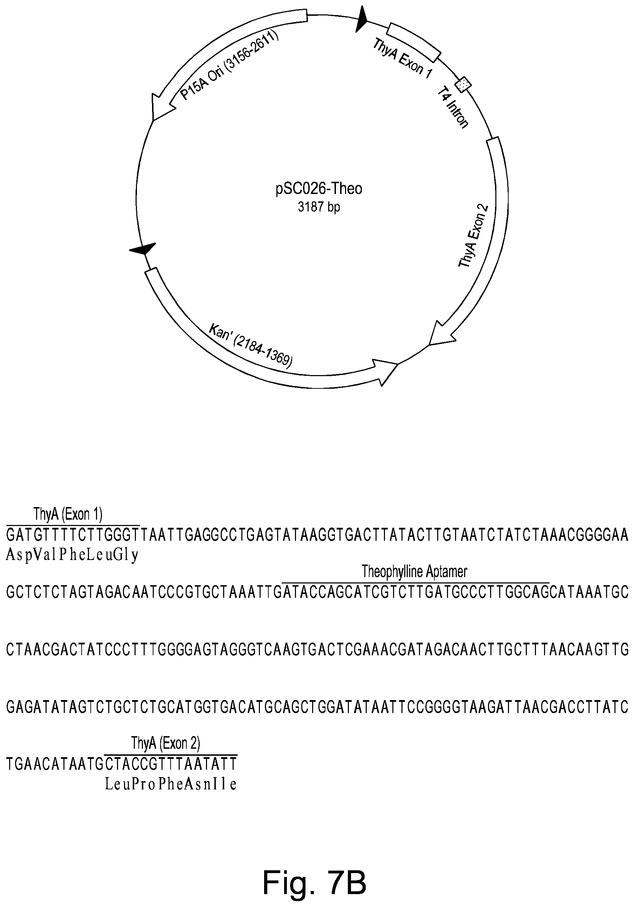

The plasmid encoded *thyA* gene may be interrupted by a theophylline responsive self-splicing intron; single or in tandem. The pSC018-Theo contains one intron in the coding sequence and does slow down the growth significantly when not induced, however during prolonged incubation (overnight) the non-induced bacteria grow to a similar density as the induced bacteria. While induction does give a growth advantage, the selection is not black and white. A single intron insertion on another position (figure 7B) yields a similar picture as an intron inserted on the wild type position. Only two introns in tandem provide enough control to have no growth at all while the bacteria is not induced and a dose dependent growth when they are

## Detailed Description

The invention will now be described in detail with reference to the examples and to the drawings in which:

**Figure 1A** shows a common known mechanism of engineered riboswitches in bacteria. For regulation of translation initiation. When there is no ligand a stem-loop is formed by the aptamer domain and by a sequence 5' to and complementary to the Shine Delgarno region, the latter being accessible to the 30S RNA for binding and therefore translation initiation occurs. When ligand binds aptamer then a folding of the aptamer domain occurs as well as an alternative stem loop structure involving the SD region. This then blocks 30S binding and stops translation initiation.

**Figure 1B** shows a regulation of transcription termination. The aptamer is fused to a short spacer region followed by a sequence complementary to the 3' part of the aptamer and a U stretch. When no ligand is present then the complementary 3' part is base paired with the aptamer forming a terminator structure so that RNA polymerase (RNAP) dissociates and transcription is blocked. When ligand binds aptamer then this terminator structure formation is inhibited and transcription can proceed.

**Figure 1C** shows an illustration of one of the mechanisms of a riboswitch located in the 5'-UTR of bacterial mRNA.

**Figure 2** shows the structures of two introns as shown in Thompson *et al.*, 2002 BMC Biotechnol **2**:21. This drawing is from © 2002 Thompson et al; licensee BioMed Central ltd. This is an Open Access article: verbatim copying and redistribution of this article are permitted in all media for any purpose, provided this notice is preserved along with the article's original URL: http://www.biomedcentral.com/1472-6750/2/21 . Panel A shows the parental self-splicing intron of phage T4 SEQ ID NO:1 (which does not need an inducer to splice). Panel B shows SEQ ID NO:10 and an engineered variant SEQ ID NO:11, in which the P6a-loop (see panel A, box) has been replaced by a theophylline-binding aptamer; binding of theophylline induces a conformational change that triggers splicing activity (see panel A, arrows indicate cleavage in loops P1 and P10).

**Figure 3** shows part of the pyrimidine synthesis pathway. dTMP is a key link in the synthesis of DNA. The pathway from thymine to dTMP is not supported by *E. coli*, while the pathway from DNA to dTMP does not support growth.

Bacteria can therefore be grown under non-selective conditions by adding thymidine (and indeed not thymine) to the medium, a compound which has a very low abundancy in common medium components such as tryptone, peptone and yeast extract.

**Figure 4** shows the relationship between growth rate of single intron thyA constructs (i.e. those with a single self-splicing intron) and promoter strength. *E. coli* DH10B-ΔthyA carrying the reporter constructs were grown with different amounts of theophylline and monitored for 20h.

**Figure 5** shows a comparison of the phage T4 td intron SEQ ID NO:12 with the mutant td intron SEQ ID NO:13. The mutant td intron differs only slightly from the phage T4 td intron in the 5' and 3' flanking sequence and retains its activity. The flanking regions which are part of the riboswitch, but not of the intron (they are part of the exons) are WT and mutated in intron 1 and 2 respectively.

**Figure 6** shows the growth rate of both single and double intron thyA constructs under control of the $P_{taci}$ promoter under varying theophylline concentrations. Intron 1 (◊) is the intron at (F171 - P175), intron 2 (△) is the intron at (H51 - I55). A construct featuring no intron in the thyA gene (□) is also shown.

**Figure 7** shows vector maps for dTMP auxotrophy complementation. Panel A shows the intron insertion SEQ ID NO:14 like in the phage T4 td intron situation. Panel B shows the introduction of the functional intron SEQ ID NO:15 upstream of the wild type position, such that the introduction is silent in the sense that no amino acids were changed in the protein sequence into which the intron is inserted. Panel C shows the tandem introns SEQ ID NO:16 for improved control of expression.

**Figure 8** shows the structure of a self-splicing intron (Cech *et al*., 1994). In loop 6a, sequences have been inserted that affect the splicing activity. Insertion of ligand-binding RNA fragments (aptamers) controls the splicing by a conformational change, triggered by the presence or absence of a specific ligand.

**Figure 9** shows the structure of a small RNA fragment SEQ ID NO:17 with an aptamer. The aptamer sequence can be varied using Systematic Evolution of Ligands by Exponential Enrichment (SELEX) and screened for novel specificities.

**Figure 10A** shows a single self-splicing intron construct.

**Figure 10B** shows a double self-splicing intron construct.

**Figure 10C** compares the structures of pSC024, pSC026, pSC018 and pSC022.

**Figure 10D** shows a schematic of the "reporter cascade" described for GFPuv production, although any protein may be produced. The T7 polymerase gene is carried on the bacterial genome. The enzyme's functionality depends on maturation (RNA splicing that restores open reading frame), which is controlled by theophylline in case of the heterologous expression system, or a compound derived from an enzymatic reaction when the system is utilised as a screening method.

**Figure 11** shows the structure of expression vectors for GFPuv expression dependent on rhamnose and theophylline.

**Figure 12** shows the vector map for the reporter plasmid pSC028-GFPuv-term. pSC028-GFPuv-term was constructed using pACYC184 as a base.

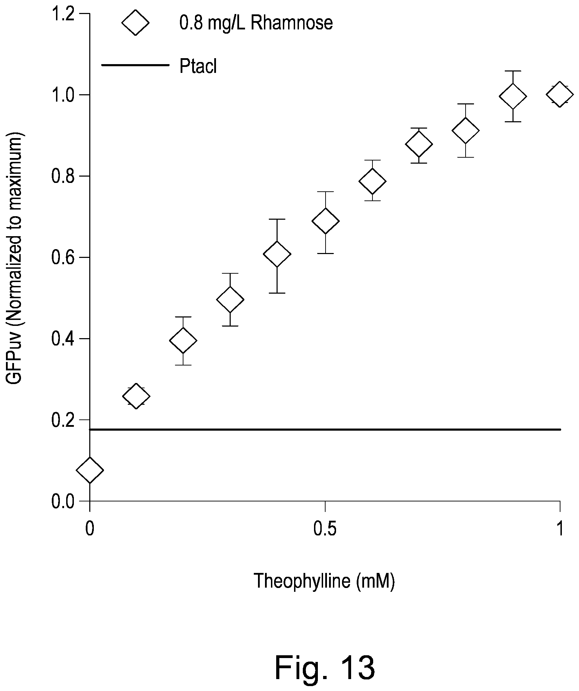

**Figure 13** shows theophylline dependency of the cascade at 0.8 mg/L rhamnose. The Ptacl serves as benchmark for a strong promoter dependent on *E. coli* RNA polymerase and is the pSC034f construct.

**Figure 14** shows expression of GFPuv over a wide range of rhamnose and theophylline concentrations.

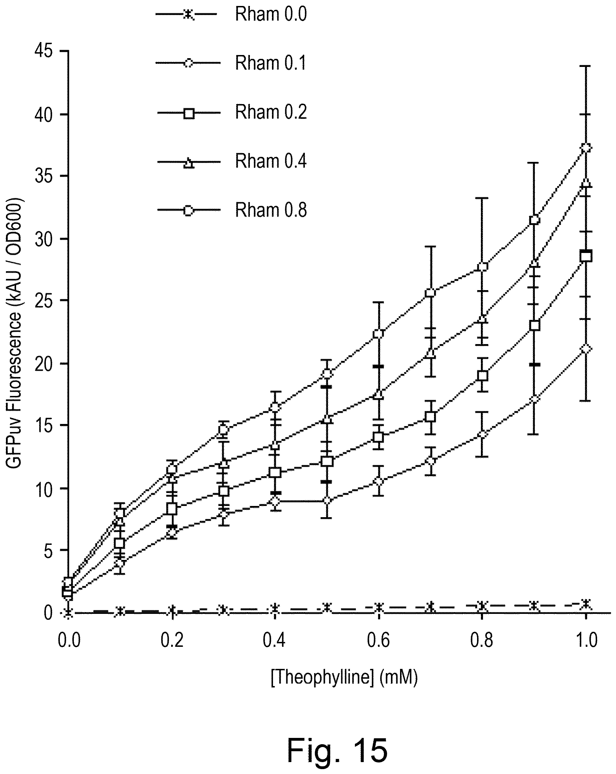

**Figure 15** shows the sensitivity of the cascade, showing the theophylline dependency of the cascade in response to differing concentrations of rhamnose. The cascade is controlled by one theophylline dependent intron at the first position depicted in Figure 10. In particular expression of GFPuv using the single intron driven cascade over a wide range of rhamnose and theophylline concentrations is shown.

**Example 1 - Evaluation of T4 Intron-controlled ThyA auxotrophy**

An *in vivo* biosensor is usually composed of a control element and a reporter gene. The reporter gene can confer antibiotic resistance, fluorescence, auxotrophy complementation or luminescence. The control element can act on several stages in the protein production process. Protein based control elements like LacI typically intervene with the transcription of the gene, while inteins and post-translational modification deal with activation of the protein itself. In between, there is the control on translational level predominantly performed by riboswitches. The riboswitches are mostly located in the 5'-UTR sequestering and releasing the Shine-Dalgarno sequence to block or allow translation by the ribosome. For example, Figure 1 shows the operation of a generalised riboswitch, illustrating how structural changes in the RNA fragment induced by the binding of a signal metabolite may result in the reduced accessibility of the Shine-Dalgarno sequence and the blockage of translation. Several cases have been described where the 5' untranslated region (5' UTR) of mRNA transcripts form secondary structures (e.g. stem-loop structures) *via* intra-molecular base paring. The structure of an RNA fragment may change upon specific binding of a ligand, and may affect the accessibility of the ribosome binding site (Shine-Dalgarno sequence) and as such the translation efficiency (riboswitch on/off). Variation of the sequence of the signal metabolite (ligand)-binding sub-fragment ('aptamer') may adjust the specificity of the riboswitch.

It may be difficult to alter the ligand of these riboswitches, since the anti-Ribosome Binding Site (anti-RBS) or anti-terminator may be part of the aptamer domain. Altering the aptamer domain will change the anti-RBS or anti-terminator rendering the riboswitch inactive. Randomising the 5'-UTR and testing for riboswitch activity is one solution to that problem. Another possibility is changing a ribozyme, either a synthetic or a natural one, into a riboswitch by attaching an aptamer domain. This creates an allosteric ribozyme also referred to as aptazyme. An aptazyme that was based on the hammerhead ribozyme was designed by Ogawa and Maeda (2007) Bioorg Med Chem Lett 17:3156-3160.) (This aptazyme is based on SD sequestering and the block is released by the endonuclease activity of the ribozyme upon induction. A different approach using the same mechanism can be applied in eukaryotes cutting of the poly-A tail upon induction. A type of synthetic riboswitch that has not been studied extensively is the group I aptazyme. This aptazyme is a modified version of a group I self-splicing intron. The intron that was modified is derived from the phage T4 *td* gene encoding thymidylate synthase (see Figure 2). This gene has a homologue in *E. coli* named *thyA.* In the engineered variant, derived from the parental self-splicing intron of phage T4 (both shown in Figure 2), the P6a-loop (see panel A, box) has been replaced by a theophylline-binding aptamer. Binding of theophylline induces a conformational change that triggers splicing activity (see panel A, arrows indicate cleavage in loops P1 and P10).
This system has many properties that make it suitable as an *in vivo* biosensor. Contrary to riboswitches that block the Ribosome Binding Site (RBS), there is only one way leakage can occur; when blocking the RBS the block may be released by complete unfolding of part of the mRNA. When the intron is unfolded it does not splice out of the mRNA, still disallowing functional translation. The leakage that will occur in both instances is when the aptamer is not completely destabilised when no ligand is present and the switch is flipped in the absence of a trigger. The gene the intron is naturally present in is an essential gene that can be complemented easily by adding thymidine to the medium or supplying the gene on a plasmid. By default, no large amount of thymidine is present in most widely used media like LB, so all experiments can be performed on rich media. The selection allows for a great number of variants to be tested without expensive equipment or labour intensive experiments. A property the *td* intron based riboswitch shares with other riboswitches is the transferability to other organisms, as riboswitches are unaffected by post-translational modification. Group I introns have the extra advantage that no species-specific elements like SD sequence or poly-A tail are involved. This experiment focuses on the exact conditions the theophylline responsive T4 *td* intron needs to function as selection tool in *E. coli.*

***Auxotrophy complementation with thyA under control* of a *theophylline dependent intron***

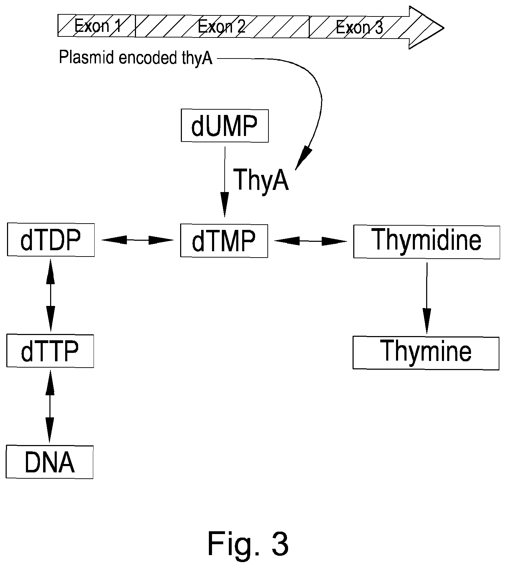

*thyA* is a gene encoding thymidylate synthase, a crucial part of the pyrimidine synthesis pathway. It catalyses the reaction from dUMP to dTMP using THF as a cofactor (see Figure 3). As a precursor of dTTP, which is required for DNA synthesis dTMP is a key link in the synthesis of DNA. The pathway from thymine to dTMP is not supported by *E. coli*, while the pathway from DNA to dTMP does not support growth. dTMP can also be derived from thymidine when this is added to the medium, but not thymine as *E. coli* DH10B lacks the enzyme to convert thymine into thymidine. The ThyA deficient bacteria cannot grow on rich medium without addition of thymidine.

The relationship between the growth rate of single intron constructs (i.e. those with a single self-splicing intron) and promoter strength was determined.

The theophylline dependent phage T4 *td* intron was designed according to Thompson et al (2002) BMC Biotechnol. 2: 21. The intron flanking regions are identical on protein level between the *thyA* gene of *E. coli* and the *td* gene of phage T4 allowing for introduction of the intron into *thyA* with silent mutations only.

Reporter constructs carry a p15A origin of replication derived from pACYC184, a kanamycin resistance gene from pET24d and the 5'-UTR and CDS of *E. coli thyA*. A terminator and promoters of different strength were placed upstream of the 5'-UTR. All promoters are listed in Table 1. *E. coli* DH10B-ΔthyA carrying the reporter constructs were grown with different amounts of theophylline and monitored for 20h.

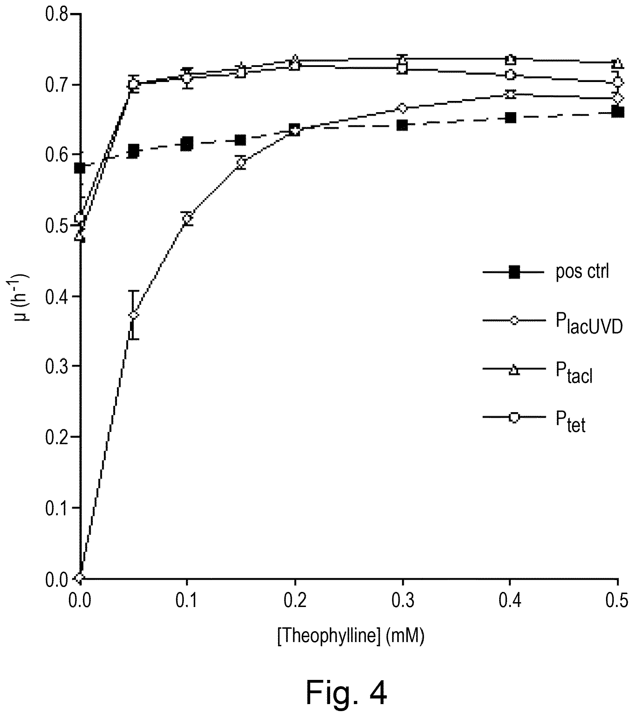

The log phase growth rate was determined in biological triplicate for each construct and theophylline concentration (Figure 4).

The constructs with promoters $P_{tacI}$ (Δ) and $P_{tet}$ (○) show near maximum growth while not being induced and maximum growth with slight induction. The $P_{lacUV5}$ (◊) construct shows the highest theophylline dependency having no growth without induction and a dynamic range of 0 mM - 0.4 mM theophylline resulting in growth rate 0 h$^{-1}$ - 0.69 h$^{-1}$. The promoters $P_{ara}$, $P_{bla}$, $P_{cat}$ and $P_{lac}$ do not support log phase growth nor does the negative control (frame shifted *thyA*). *E. coli* DH10B containing the frame-shifted *thyA* serves as positive control (closed square) (Figure 4).

**Table 1.** Promoter sequences used in the expression of reporter constructs in *E. coli*

| Code | Name | Sequence -35box                          -10box | |
|---|---|---|---|
| Consensus seq | | TTGACANNNNNNNNNNNNNNNNNN-TATAAT | SEQ ID NO:1 |
| SC019a | P_ara | CTGACGCTTTTTATCGCAACTC--TCTACT | SEQ ID NO:2 |
| SC019b | P_bla | TTCAAATATGTATCCGCTCATGA-GACAAT | SEQ ID NO:3 |
| SC019c | P_cat | ATGAAATAAGATCACTACCGGGCGTATTTT | SEQ ID NO:4 |
| SC019d | P_lac | TTTACACTTTATGCTTCCGGCTCGTATGTT | SEQ ID NO:5 |
| SC019e | P_lacUV5 | TTTACACTTTATGCTTCCGGCTCGTATAAT | SEQ ID NO:6 |
| SC019f | P_tacI | TTGACAATTAATCATCGGCTCG--TATAAT | SEQ ID NO:7 |
| SC019g | P_tet | TTGACAGCTTATCATCGATAAGC-TTTAAT | SEQ ID NO:8 |

In all cases a higher ThyA expression results in more growth. More growth does not necessarily mean that the ThyA expression is high enough to sustain the growth. No growth above OD$_{600}$ of 0.040 was observed for bacteria carrying *thyA* under control of the $P_{ara}$, $P_{bla}$, $P_{cat}$ and $P_{lac}$ promoters. These promoters do not support log phase growth, but can extend the period the bacteria can grow on the carry-over thymidine depending on the promoter strength and induction by theophylline.

Theophylline dependent log phase growth was observed for $P_{lacUV5}$, $P_{tacI}$ and $P_{tet}$. These promoters more closely resemble the consensus sequence of the -35 and -10 regions. To observe better growth when the auxotrophy complementation is under control of a stronger promoter is to be expected. The promoters $P_{tacI}$ and $P_{tet}$ are strong enough to support log phase growth without induction by theophylline, while the $P_{lacUV5}$ promoter does not. The positive control, *E. coli* DH10B with the frame-shifted *thyA* gene, appears to be slightly theophylline dependent. This effect is marginal and is most likely caused by the position the samples have on the microtiter plate rather than the theophylline concentration (Figure 4).

Auxotrophy complementation indirectly depends on the concentration of mature mRNA. The concentration of mature mRNA depends on the concentration of immature mRNA and the maturation rate. The concentration of immature mRNA is mostly dependent on promoter strength, while the maturation rate is dependent on theophylline induction. The maturation rate does not equal zero when the no inducer is present. This leakage is shown by the constructs having a strong promoter in front of the coding sequence. Where there no leakage, promoter strength should have no effect when no inducer is present. The weak promoter constructs do not generate enough mature mRNA even when the maturation rate is high. The amount of ThyA is not enough to reach the minimal concentration of dTTP required in the cell. A concentration below the minimal requirement will result in thymineless death. It appears there is a fine line between never enough ThyA and always enough ThyA. The balance is matched rather well with the $P_{lacUV}$ promoter. No growth is observed when no inducer is present and maximum growth is observed at full induction (Figure 4).

Although the growth of *E. coli* DH10B-ΔthyA carrying the $P_{lacUV5}$ construct was not observed in microtiter plates, it sometimes was observed in 5 mL cultures in a 50 mL Greiner tube. Evaporation is a serious issue in the microtiter plate only causing problems after several hours of growth. By that time all exponential growth was finished already and carry-over thymidine was consumed staggering the growth. The bacteria in the Greiner tube did not suffer from evaporation, so a very small subpopulation having slightly increased expression may become dominant overnight indicating that the background expression of ThyA is only just below the minimal requirement, sometimes exceeding it. While this background growth may not be interfering with competition experiments between induced and uninduced bacteria, it may lead to false negatives.

**Example 2** - **Introduction of a second intron into the thyA coding sequence**

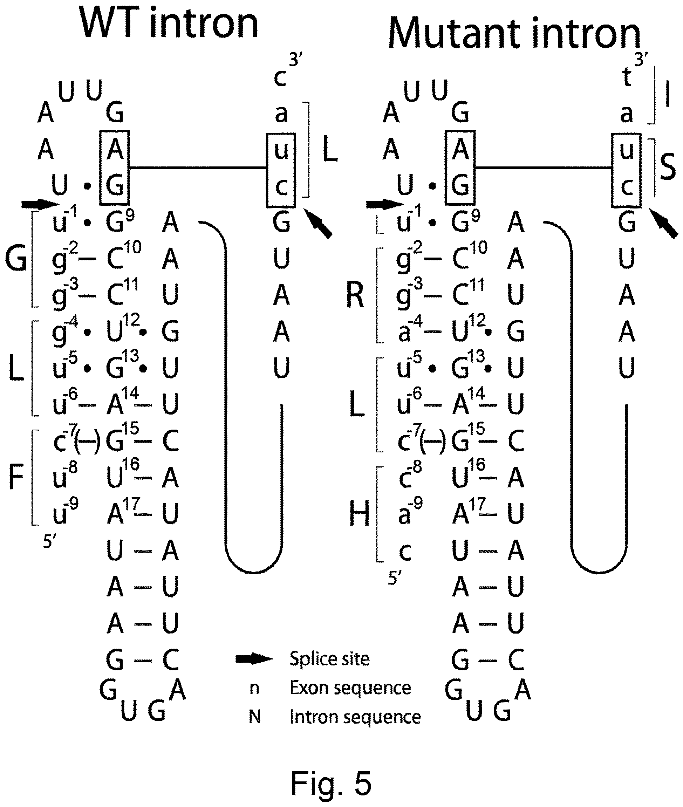

The strong promoters $P_{tacI}$ and $P_{tet}$ showed leakage exceeding the minimal requirement of ThyA (Figure 4). A second intron was introduced into the coding region of *thyA*. No other part of the *thyA* gene matches the native flanking regions of the phage T4 *td* intron, so another strategy was applied. The absence of an easily identified insertion site presents a challenge as the intron ribozyme is composed not only of the intron region itself, but of the 3' flank of exon 1 and the 5' flank of exon 2 as well. The intron flanks are therefore part of both the ribozyme and the coding region (Figure 5).

Previously, Pichler et al. (Pichler and Schroeder, 2002, J Biol Chem 277:17987-17993) showed that the flanking sequence does not need to match the native flanking sequence perfectly for a functional phage T4 *td* intron. Next to some tolerance in the intron flanking regions, the coding sequence can be composed of different codons. An algorithm was written to analyse the *thyA* coding sequence for possible locations for the intron to be inserted. The position had to match several requirements: 1) Mutations in the coding sequence were to be translationally silent for both the intron flanking regions and the restriction sites to clone the second intron into the *thyA* gene; 2) The flanking regions of the second intron had to match the flanking regions of the native intron as closely as possible; 3) No mutations in the flanking regions were allowed other than described by Pichler et al. (Pichler and Schroeder, 2002, J Biol Chem 277:17987-17993); and 4) Possibility of silent introduction a restriction site next to either flank was preferential.

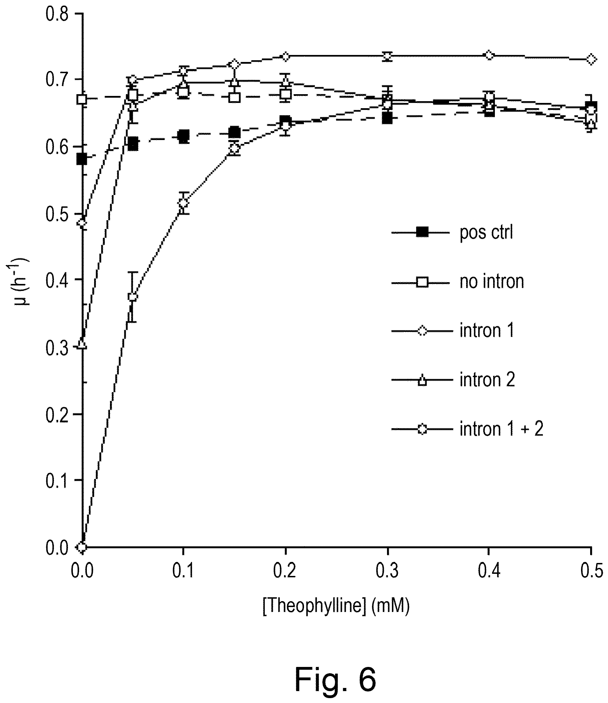

The top candidate position was identified as HLRSI (amino acids 51-55) with the intron in frame 2 and only one mutation in the intron flanking region changing a wobble base pair into a U-A base pair. Two unique restriction sites could be mutated close to the insertion site: Psp1406I upstream and PstI downstream. A construct with a tandem intron at (H51 - I55) and (F171 - P175) and a construct with the (H51 - I55) intron only were made. In both cases, the *thyA* gene was under control of the $P_{tacI}$ promoter. The constructs were tested in *E. coli* DH10B-ΔthyA according to the same protocol as the single intron constructs (Figure 6).

The growth rate of both single and double intron constructs under control of the $P_{tacI}$ promoter was measured (Figure 6). Intron 1 (◊) is the intron at (F171 - P175), intron 2 (Δ) is the intron at (H51 - I55). Introns 1 and 2 in tandem (○) shows a theophylline dependency with a dynamic range of 0 mM - 0.4 mM theophylline resulting in a growth rate of 0 $h^{-1}$ - 0.67 $h^{-1}$. Less leakage is observed with Intron 2, but less maximum growth rate as well, indicating a lower splice rate. No intron in the *thyA* gene (□) results in maximum growth regardless of the theophylline concentration.

A reduction of background was discovered with the second intron (Figure 6). The tandem intron completely erases the background growth even with the $P_{tacI}$ promoter. Aside from the absence of background growth on microtiter plate, no bacterial growth was observed lacking both thymidine and theophylline. The second intron at (H51 - I55) alone shows a slightly lower ThyA expression compared to the first intron at (F171 - P175). This result may be caused by the difference in position, the difference in sequence or both. The difference in position means that the surrounding parts of the mRNA will have different secondary and tertiary structures as well as a different translation speed. This may affect the splicing rate of the intron. The difference in sequence will affect the ThyA production in two ways. Firstly, the intron splice rate is directly dependent on the intron flanking region (Pichler and Schroeder, 2002, J Biol Chem 277:17987-17993) which is not the same for intron 1 and 2. Furthermore, by introducing the silent mutations for the restriction sites and the intron flanking region, the amino acid sequence may not be altered, but the codon usage is. The difference in codon usage may influence the ThyA expression either in a positive or negative way.

The phage T4 *td* intron is therefore a useful tool for selection of *E. coli* that have a small molecule inside their plasma membrane. The ability of this system to completely select against bacteria that have no such small molecule present makes it relatively straightforward to select for the bacteria that do. Leakage and fully-induced expression can be carefully adjusted so bacteria without small molecule do not grow at all, whilst the bacteria with small molecule do. It was shown that the $P_{lacUV5}$ promoter can balance the leakage and the induced expression so that the dynamic range is between 0 mM and 0.4 mM theophylline resulting in a growth rate between 0 $h^{-1}$ and 0.69 $h^{-1}$ on microtiter plate. However, this particular promoter is not expected to support Log phase growth and is not so preferred. A direct route to manage balance between leakage and full expression is the introduction of a second intron at an upstream position. Tandem introns are significantly more effective in reducing background splicing, while maintaining the dynamic range in both inducer concentration and growth rate.

**Materials and methods**

*Chemicals and plasmids*

Thymidine and theophylline were purchased from Sigma-Aldrich (St. Louis, MO). A plasmid containing the E. coli *thyA* gene interrupted by a modified phage T4 *td* intron between G173 and L174 was commissioned at GeneArt (pMA-ThyA-SI001) as well as an intron version containing a theophylline responsive aptamer (pMA-ThyA-Theo). Plasmid pET24d was purchased from Novagen. Plasmid pRham C-His was purchased from Lucigen.

Enzymes were purchased from Thermo Scientific and used according to the manufacturer's instructions, unless stated otherwise.

*Bacterial strains and media*

*E. coli* DH10B T1[R] was purchased from Invitrogen (C6400-03) and used for plasmid propagation and standard molecular techniques, as well as a parent strain for the *thyA* deficient *E. coli* DH10B-ΔthyA strain. Transformation was performed with a ECM 63 electroporator (BTX) at 2500 V, 200 Ω and 25 μF, 2 mm cuvettes, 20-40 μL of electro-competent cells and recovery in LB.

Bacteria were generally grown at 37°C on LB medium (Miller) containing the appropriate antibiotics: kanamycin (50 mg/L), ampicillin (100 mg/L), chloramphenicol (35 mg/L) and tetracycline (15 mg/L). In addition, the auxotrophic *E. coli* DH10B-ΔthyA was complemented with thymidine (100 mg/L) when necessary.

*Construction of reporter plasmids*

The reporter plasmids pSC018a-g - Theo were constructed using pACYC184 as a base. The steps include exchange of the chloramphenicol acetyltransferase (*cat*) for the aminoglycoside 3'-phosphotransferase (*kan*) from pET24d (Novagen), exchanging the *Tet*A(C) for the *thyA* gene encoded on the pMA-ThyA-SI001 plasmid and exchanging the 6b hairpin for the theophylline responsive aptamer from pMA-ThyA-Theo).

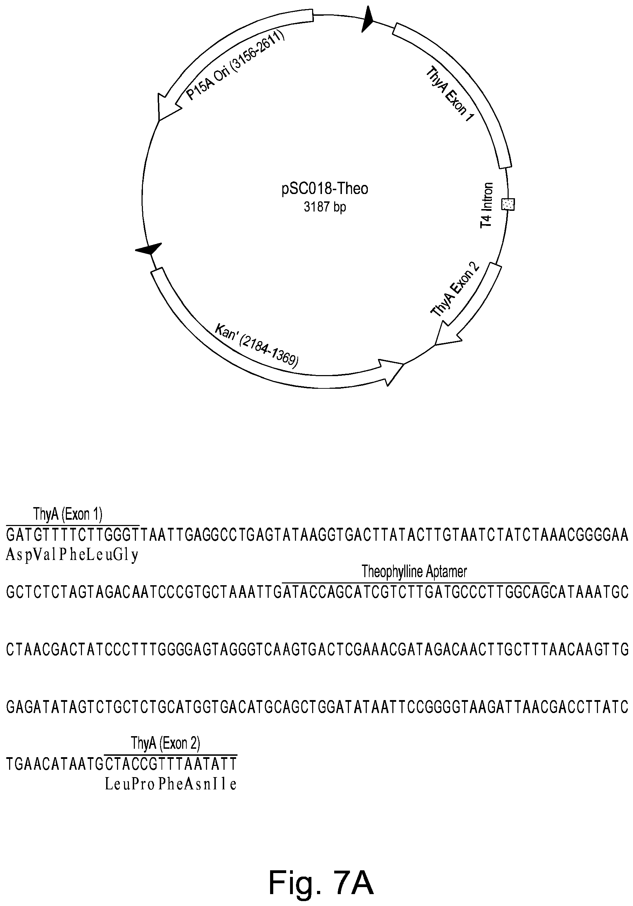

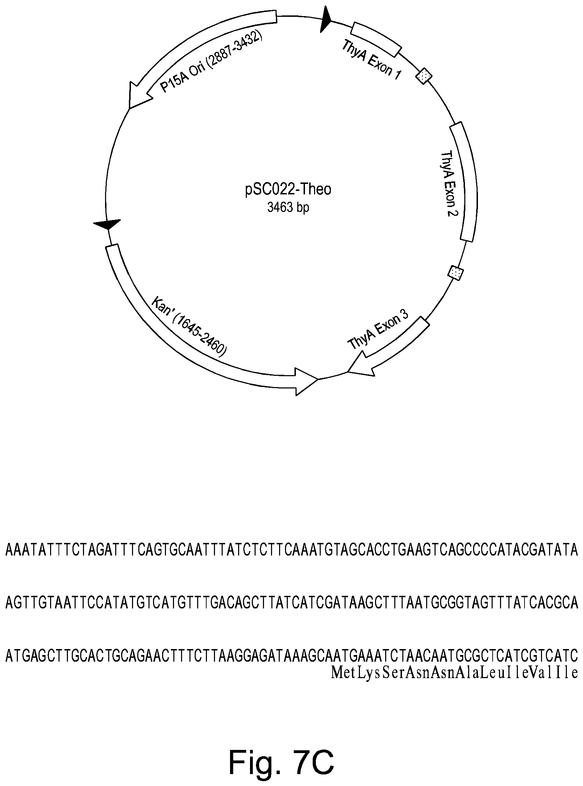

Promoter variants were made by polymerase chain reaction (PCR) and ligating the PCR product into pSC018f-Theo (Figure 7A) between the KpnI and BcuI sites. pSC022f-Theo (Figure 7C) was constructed by cloning a second theophylline responsive intron into pSC018f-Theo between R53 and S54 using the Psp1406I and PstI sites. The second intron was generated by PCR using pMA-ThyA-Theo as a template. pSC024f and pSC026f-Theo (Figure 7B) were constructed by using pSC018f-Theo and pSC022f-Theo respectively as template for PCR. Ligation of the PCR products into pSC018f between the Acc65I and MluI site removed the intron between G173 and L174, leaving no intron in pSC024f and one intron between R53 and S54 in pSC026f-Theo. A frame-shift construct in the *thyA* gene of pSC024f was constructed by digestion with MluI, Klenow fragment 5' fill-in and re-ligation of the plasmid.

DNA purification was performed with the DNA Clean & Concentrator-5 kit of Zymo Research (D4004) or the Zymoclean™ Gel DNA Recovery Kit (D4002). Plasmid was isolated with the Plasmid Miniprep kit of Thermo Scientific (#K0503). Ligation was performed at 22°C for 1h, followed by 10 min heat inactivation. All plasmids were verified by PCR and/or restriction analysis and sequencing by GATC Biotech (Konstanz, Germany).

*Construction of the thymidine synthase deficient strain*

The *thyA* deficient strain DH10B-ΔthyA was made according to a standard protocol (Datsenko and Wanner (2000) PNAS 97: 6640-6645) with the exception of the PCR template and the competent cells protocol and the PCR template for the insertion cassette.

Electro-competent cells were made by growing DH10B T1[R] (Invitrogen) containing pKD46 at 30°C on 16 g/L peptone, 10 g/L yeast extract and appropriate antibiotic to an $OD_{600}$ of 0.4 and cooled down to 4°C, washed with ultrapure water once and 10% glycerol twice. Finally the bacteria were concentrated 250x in 10% glycerol.

DH10B T1[R] containing pKD46 was transformed with a PCR product generated from pMA-RQ-Lox71-kan-Lox66, kindly provided by Teunke van Rossum, containing a kanamycin resistance gene flanked by Lox71 and Lox66. The Lox sites can be recombined by cre recombinase removing kanamycin resistance, but do not form a functional Lox site. Transformed bacteria were recovered in LB medium containing thymidine (100 mg/L) for 2.5 h at 37°C and plated on LB agar plates containing kanamycin (50 mg/L) and thymidine (20 mg/L). Colonies were verified for *thyA* deficiency by plating on LB agar plates containing kanamycin (50 mg/L). Plasmid curation was assessed by growing on LB agar plates containing ampicillin (100 mg/L) and thymidine (20 g/L).

Electro-competent cells were made from DH10B T1[R] -ΔthyA-kan growing on medium containing kanamycin (50 mg/L) and thymidine (100 mg/L) at 37°C and transformed with pJW168 containing the cre recombinase. Auxotrophy, recombination of the Lox sites and plasmid curation were assessed by plating on LB agar medium, LB agar containing kanamycin (50 mg/L) and thymidine (20 mg/L) and plating on LB agar medium containing ampicillin (50 mg/L) and thymidine (20 mg/L). Electro-competent cells were made of the knock-out strain and transformed with the auxotrophy reporter constructs.

*E. coli DH10B-ΔthyA growth assays*

*E. coli DH10B-ΔthyA* containing a reporter construct of the pSC series were grown overnight at 37°C on LB medium containing kanamycin (50 mg/L) and thymidine (100 mg/L). A $10^{-4}$ dilution was made and grown in with a variable amount of theophylline in a 96 well microtiter plate (Greiner) in a final volume of 200 μL. Culture plates were incubated under continuous shaking for 20h at 37°C and the $OD_{600}$ was measured every 10 minutes in a Synergy MX plate reader. As carry-over thymidine allows the knock-out strain to grow without ThyA, a lower limit $OD_{600}$ was set to 0.040 AU to negate false positive growth. Growth rate ($\mu$) was calculated from at least 1h of log phase growth exceeding an $OD_{600}$ of 0.040 according to

$$\ln(C) = \ln(C_0) \cdot \mu \cdot t$$

## Example 3 - Development of a synthetic riboswitch-ribozyme hybrid

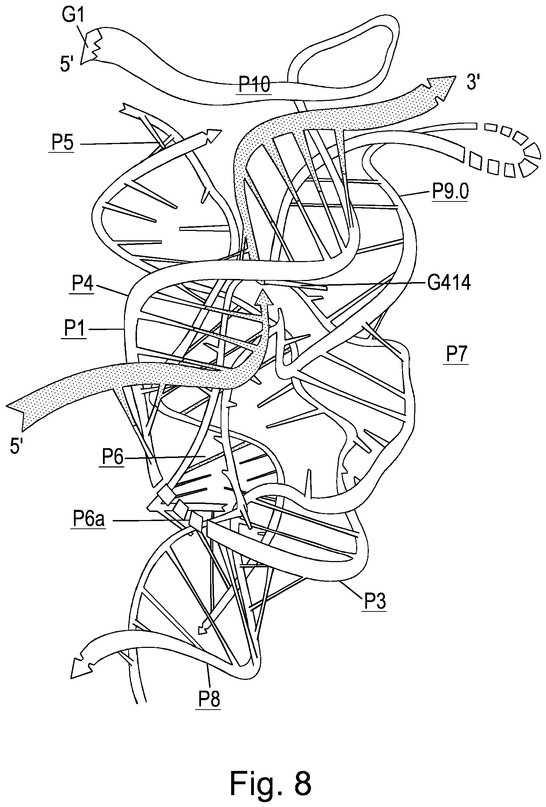

Group I self-splicing introns are RNA molecules with catalytic activity: i.e. RNA ribozymes. These introns catalyze their own excision from precursors such as mRNA. The well characterized T4 self-splicing intron has been demonstrated to adopt a specific 3D-structure that is required for catalytic activity (Figure 8). In loop 6a, sequences have been inserted that affect the splicing activity. Insertion of ligand-binding RNA fragments (aptamers) controls the splicing by a conformational change, triggered by the presence or absence of a specific ligand.

The T4 self-splicing intron has been engineered into a functional catalytic riboswitch by inserting a theophylline-binding aptamer (Figure 5). The recombinant riboswitch was still able to splice itself, but its cleavage activity was triggered by a conformational change upon binding of the aptamer ligand, i.e. theophylline (Thompson et al. 2002 BMC Biotechnol. 2: 21). The molecular mechanism of the ligand-dependent riboswitch activity is the reversible disruption of the RNA structure (Figure 5). Replacing the stem-loop by an aptamer destabilizes its formation, but the stem-loop can be restored by a ligand binding to the aptamer. However, the formation of the stem-loop can occur without binding of the ligand. The stem-loop without the bound ligand is less stable than the ligand bound stem-loop, but can cause background provided the screening or selection method is sensitive enough.

To test for riboswitch functionality, the intron/aptamer fusion was integrated in a reporter gene. Next to the aforementioned antibiotic resistance marker, also auxotrophic markers can be used (essential genes for amino acids or nucleotides are deleted in microbial hosts; growth in the absence of these amino acids or nucleotides is only possible when the corresponding gene is complemented in a plasmid). For the riboswitch test, an *E. coli* thyA knockout strain; the thyA gene encodes an essential enzyme in biosynthesis of thymidine (one of the four bases of DNA nucleotides). The thymidine auxotrophy is complemented by a plasmid-borne thyA gene. When using a plasmid with ThyA that was interrupted by the hybrid riboswitch, it was demonstrated that the thyA knockout strain of *E. coli* could survive on minimal medium containing theophylline (without thymidine), but not on minimal medium lacking both theophylline and thymidine (Thompson et al. 2002 BMC Biotechnol. 2: 21). Hence, the presence of the riboswitch ligand theophylline, allows for growth.

## Example 4 - Tandem riboswitch provides improved stringency

In the aforementioned experiments (Thompson et al. 2002 BMC Biotechnol. 2: 21), there was a background level cell growth was observed in the absence of the theophylline ligand (Figure). This background was subtracted from the growth observed in the presence of the ligand. Theophylline-induced growth was approximately 40% of the parental intron, so maximally 40% of the wild type growth. In an experiment based on the latter publication, the induction of the thyA gene by theophylline turned out to be far from stringent. Non-induced splicing of the intron was thought to be the cause of growth on minimal medium lacking both theophylline and thymidine. As a solution to the problem of non-induced splicing, a suitable second insertion site was identified, for the insertion of a second self-splicing intron.

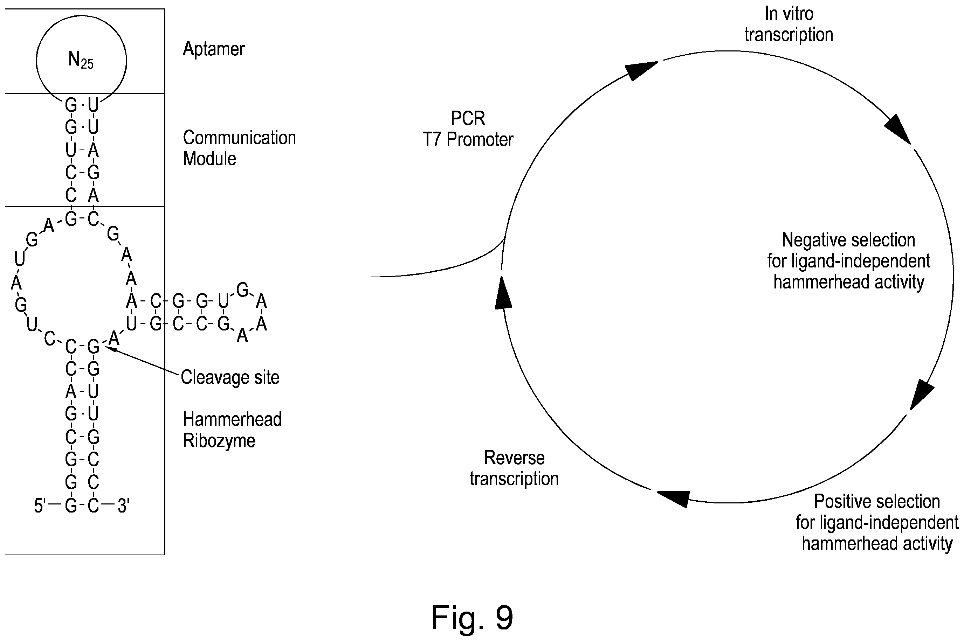

A comparison of a single intron construct with a construct of the present invention featuring a tandem self-splicing intron was made (Figure 9). Growth (OD600) was plotted against time (minutes) in the presence (1 mM) and absence (0 mM) of theophylline for the construct designed by Thompson *et al.* (Thompson et al. 2002 BMC Biotechnol. 2: 21) utilising one theophylline-dependent intron in thyA, in a thyA-deficient *E. coli* DH10B strain (Figure 9A). On this time scale the theophylline-induced bacteria outcompeted the non-induced bacteria with ease, but the non-induced bacteria continued growth until the OD600 reached a value comparable to the induced bacteria overnight (not shown). Growth was measured in the same way for a construct containing an *E. coli* thyA gene interrupted by two theophylline-dependent introns, which was transformed into a thyA-deficient *E. coli* DH10B strain. The overnight growth was plotted against the theophylline concentration in the medium (Figure 9B). The growth shows a non-linear relationship with theophylline concentration. The line drawn through the first five points represents a Michaelis-Menten-like kinetics profile with an apparent kd value of 0.1-0.2 mM theophylline (Figure 9B). Surprisingly, the absence of growth in the absence of theophylline indicates a selection method with no detectable background (Figure 9B) in contrast with the prior art construct (Figure 9A) where a low level of cell growth continues in the absence of theophylline). Above 1 mM, theophylline became slightly toxic.

The introduction of a "tandem-riboswitch" resulted in a 70% recovery of growth compared to wild type with theophylline induction, while no growth at all was observed without induction by theophylline, i.e. black and white selection.

Each mRNA only possesses a single 5' UTR and this limits the potential positions for riboswitches to be inserted. It has been shown that the introduction of more than one riboswitch can confer improved stringency on the activity of the riboswitch, by removing background levels of non-induced splicing (and therefore expression). As there is only one 5' UTR and multiple positions for an intron, instead of inserting the riboswitch in the 5' UTR, use of the coding sequence allows multiple riboswitches to be inserted into the coding sequence and therefore improved flexibility and stringency of the engineered switch. The presence of a specific ligand (e.g. theophylline) controls self-splicing of the riboswitch, thereby restoring the reading frame of the gene encoding the desired gene product.

## Example 5 - Auxotrophy complementation

Auxotrophy complementation is based on interruption of an important step in the pyrimidine synthesis pathway (Figure 3). The *thyA* gene is knocked out in the host strain *E. coli* K12 substrain DH10B and complemented by a either a plasmid encoded *thyA* copy or thymidine supplement in the growth medium.

The plasmid encoded *thyA* gene is interrupted by a theophylline responsive self-splicing intron; single or in tandem. The vector maps are depicted in Figure 7. The pSC018-Theo (Figure 7A) contains one intron in the coding sequence and does slow down the growth significantly when not induced, however during prolonged incubation (overnight) the non-induced bacteria grow to the a similar density as the induced bacteria. While induction does give a growth advantage, the selection is not black and white. A single intron insertion on another position (Figure 7B) yields a similar picture as an intron inserted on the wild type position. Only two introns in tandem (Figure 7C) provide enough control to have no growth at all while the bacteria are not induced and a dose dependent growth when they are.

## Example 6 - Intron controlled expression cascade

Being an enzyme, a single molecule of ThyA can convert a vast amount of dUMP to dTMP, thereby enhancing the signal. For other reporters, like GFPuv, this is not the case as the bacteria are only as fluorescent as there are GFPuv molecules. Signal below the detection limit is a problem in case of GFPuv as is illustrated in Table 2. The fluorescence of the bacteria themselves (pSC012) is in the same order of magnitude as the induction independent self-splicing intron (pSC034f-SI001). The induction by theophylline is not significantly observed in pSC034f-Theo. Possibly there is a difference in expression between the induction independent self-splicing intron and the non-induced and induced theophylline dependent intron, but it cannot be concluded from these data. When no intron is present in the GFPuv (pSC034f) the fluorescence is multiple orders of magnitude higher than the background fluorescence.

**Table 2 - Direct interruption of GFPuv**

| ID | GFPuv | Intron | Induction dependent | Induced | Fluorescence (AU/OD) |
|---|---|---|---|---|---|
| pSC012 | - | - | - | - | 23 |
| pSC034f | + | - | - | - | 7204 |
| pSC034f-SI001 | + | + | - | - | 33 |
| pSC034f-Theo | + | + | + | - | 16 |
| pSC034f-Theo | + | + | + | + | 25 |

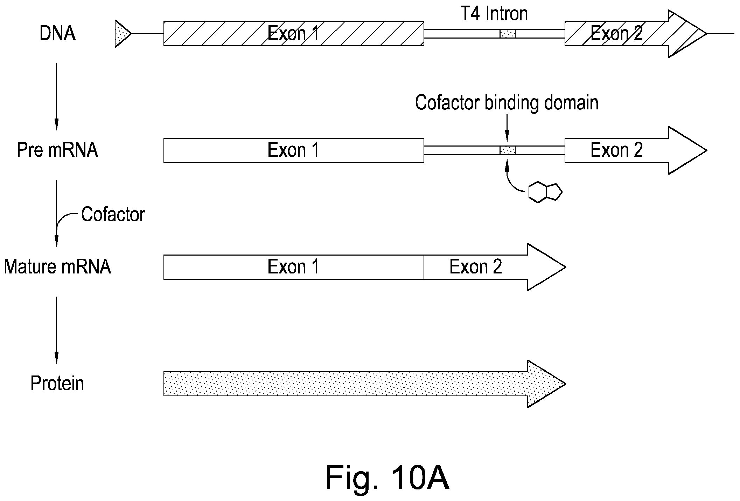

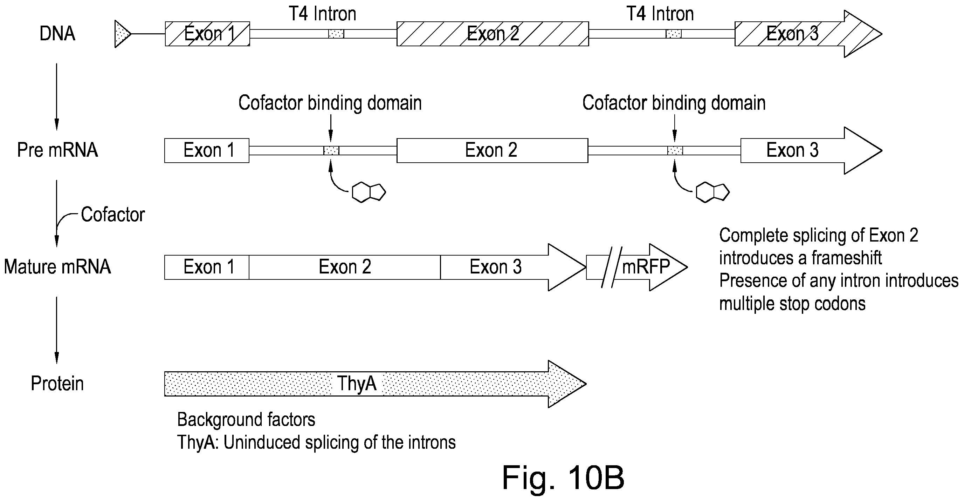

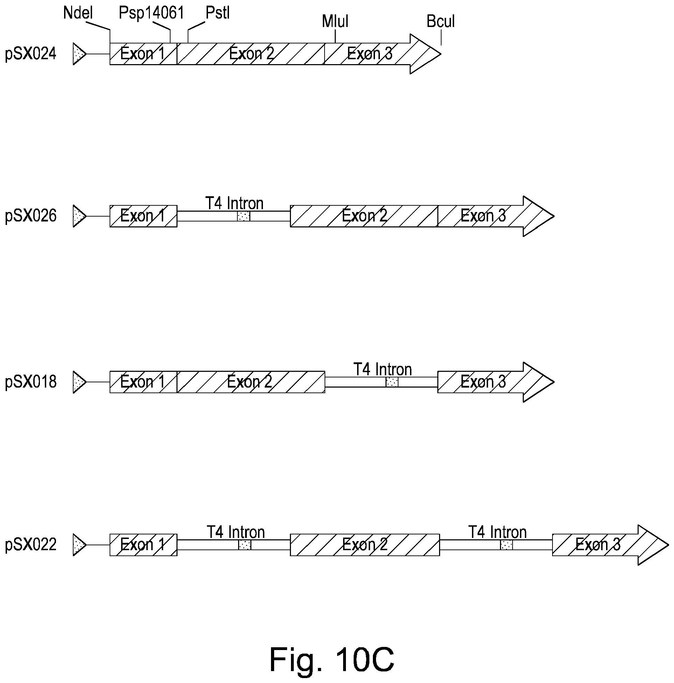

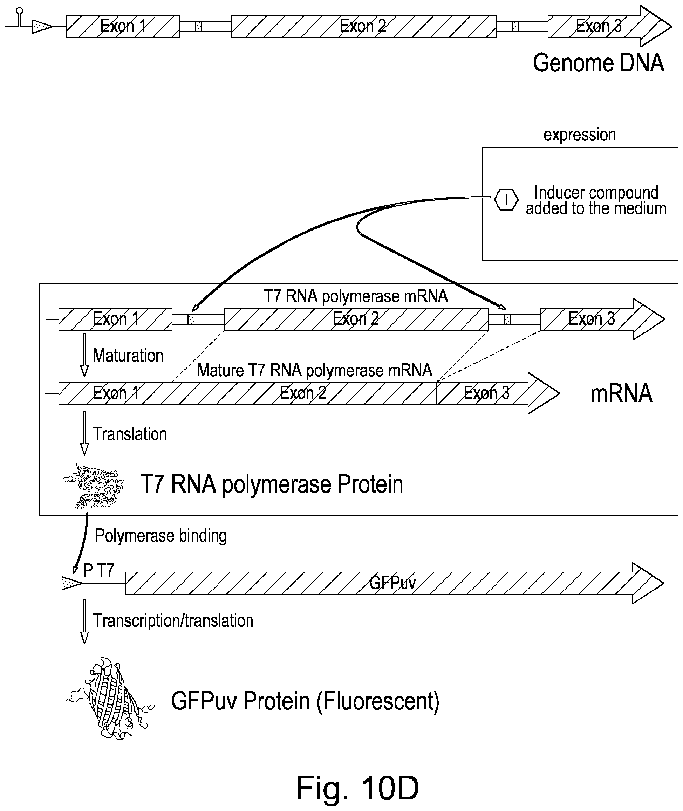

To enhance the signal from GFPuv, a cascade was made using DNA dependent RNA polymerase from phage T7. The GFPuv expression is controlled by T7 polymerase and the T7 polymerase is in turn controlled by the theophylline responsive intron (Figure 10). The T7 polymerase gene is carried on the bacterial genome. The enzyme's functionality depends on maturation, which is controlled by theophylline in case of the heterologous expression system, or a compound derived from an enzymatic reaction when the system is utilised as a screening method.

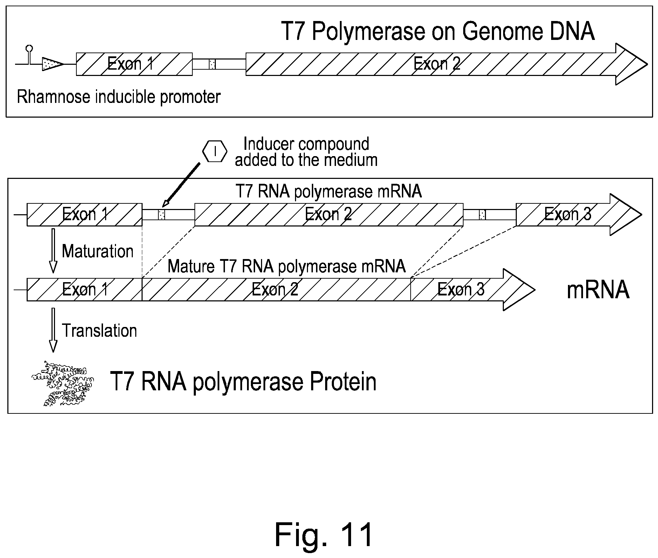

A few copies of the T7 polymerase will result in a myriad of GFPuv molecules, so a small change in T7 polymerase concentration will result in a large change in GFPuv concentration, which can be measured. Since the T7 polymerase is a very processive enzyme, it needs a tight control of expression. Figure 11 shows the polymerase is controlled on the transcription level (L-rhamnose dependent promoter) and translation level (theophylline dependent intron). Read-through GFPuv expression is reduced by an upstream terminator.

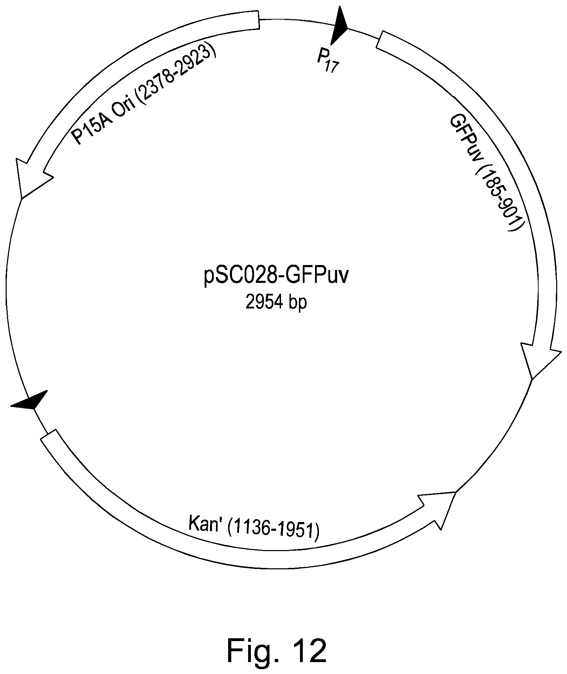

The reporter plasmid pSC028-GFPuv-term was constructed using pACYC184 as a base (vector map shown in Figure 12). The cat gene conferring chloramphenicol resistance was exchanged with the kanamycin resistance gene from pET24d. The tetA(C) gene was replaced causing an insertion site flanked by Acc65I and BcuI. The pGFPuv NdeI⁻

XhoI⁻ was used as template for a polymerase chain reaction (PCR) adding an NdeI site to the 5'-end of the GFPuv gene and a BcuI site to the 3'-end. The PCR fragment was ligated to pRham-CHis (Lucigen) digested with NdeI. This ligation added the 5'-UTR to the GFPuv gene. A PCR was performed on the ligation reaction adding an Acc65I site, a terminator and PT7 promoter in front of the 5'-UTR and a BcuI site to the 3'-end of the GFPuv gene. The secondary PCR fragment was digested with Acc65I and BcuI and ligated into the Acc65I/BcuI insertion site. Finally, the T7 terminator from pET24d was amplified by PCR adding a BcuI site to the 5'-end and an XbaI site on the 3'-end. The terminator was ligated into the BcuI site 3' of the GFPuv gene, causing the BcuI site to persist on the 5'-end of the terminator and to be removed on the 3'-end of the terminator.

The performance of the cascade was measured using GFPuv expression (Figure 12). Specifically, the theophylline dependency of the cascade at 0.8 mg/L rhamnose was measured. The PtacI serves as benchmark for a strong promoter dependent on *E. coli* RNA polymerase and is the pSC034f construct. Additionally, expression of GFPuv over a wide range of rhamnose and theophylline concentrations was determined. The cascade is controlled by one theophylline dependent intron at the first position depicted in Figure 12. The system is virtually off when either rhamnose or theophylline is absent, although the absence of L-rhamnose is more important than the absence of theophylline. The background observed when fully induced with rhamnose is about 7.5% of the maximum expression. Also there is a correlation between the background expression in the absence of one inducer and the expression level upon induction. There is a good dose-dependent relationship and the fully induced system yields a signal 5-6 times the signal observed from the strong TacI promoter. A small amount of theophylline causes a measurable signal already, which can be distinguished by techniques like FACS to separate the bacteria producing the enzyme of interest from the rest.

It is unlikely that an enzyme of interest will provide a concentration as high as 1 mM for every small molecule that is screened. As the enzyme of interest functions inside the cell, the cell membranes acting as a barrier will help the production of GFPuv rather than diminishing it. Usually aptamers have a dissociation constant in the low $\mu$M range, so for maximum signal the intracellular theophylline concentration does not have to be 1 mM, but much less.

## Example 8 - Amplification of the signal using a T7 GFPuv Cascade

### *Construction of reporter plasmids*

The reporter plasmid pSC028-GFPuv-term (Figure 12) was constructed using pACYC184 as a base. The *cat* gene conferring chloramphenicol resistance was exchanged with the kanamycin resistance gene from pET24d. The *tetA*(C) gene was replaced causing an insertion site flanked by Acc65I and BcuI. The pGFPuv NdeI⁻ XhoI⁻ was used as template for a polymerase chain reaction (PCR) adding an NdeI site to the 5'-end of the GFPuv gene and a BcuI site to the 3'-end. The PCR fragment was ligated to pRham-CHis (Lucigen) digested with NdeI. This ligation adds the 5'-UTR to the GFPuv gene. A PCR was performed on the ligation reaction adding an Acc65I site, a terminator and $P_{T7}$ promoter in front of the 5'-UTR and a BcuI site to the 3'-end of the GFPuv gene. The secondary PCR fragment was digested with Acc65I and BcuI and ligated into the Acc65I/BcuI insertion site. Finally, the T7 terminator from pET24d was amplified by PCR adding a BcuI site to the 5'-end and an XbaI site on the 3'-end. The terminator was ligated into the BcuI site 3' of the GFPuv gene, causing the BcuI site to persist on the 5'-end of the terminator and to be removed on the 3'-end of the terminator.

The plasmid pRham-CHis (Lucigen) was used as base for constructing the T7 polymerase variants. The CDS is flanked by an NdeI site and 6xHis tag on the 5'-end and a BglII site on the 3'-end. The intron positions are between G201 and L202 flanked by PscI and HindIII, between G449 and L450 flanked by Bsu15I and XagI and between G671 and L672 flanked by Eco88I and PstI. All have CAAGGGT as 5' intron flank instead of wild type CTTGGGT. The 3' intron flanks are CTAC, CTAC and CTAA respectively.

### *GFPuv fluorescence*

*E. coli* DH10B-T7His-Theo4 was grown overnight at 37°C in LB medium containing kanamycin (50 mg/L). A 96 well 2 mL culture plate (Greiner) was filled with a concentrate of theophylline and L-rhamnose. LB medium containing kanamycin and overnight grown bacteria were added so that the final concentration of kanamycin was 50 mg/L, the bacteria had a final dilution of $10^{-3}$ and the theophylline and L-rhamnose were diluted to 1x in 500 $\mu$L total volume. Culture plates were incubated at 37°C overnight under continuous shaking. The bacteria were centrifuged for 10 minutes at 4700 rpm in a Sorval Legend centrifuge. The supernatant was cleared and the cell pellet was resuspended in 500 $\mu$L 50 mM Tris-HCl pH 7.5. After resuspension, the plates were incubated at 37°C for 1 hour to allow maturation of the GFPuv. 100 $\mu$L of suspension was pipetted into a 96 well black plate with clear bottom (Perkin Elmer) and measured with a Synergy MX plate reader. The cell density was measured by scattering at 600 nm and the fluorescence was measured at an excitation wavelength of 385 nm with a width of 20 nm and an emission wavelength of 508 nm with 20 nm width with a gain of 50. The background fluorescence and background scattering were subtracted and the fluorescence was divided by the scattering at 600 nm. The background fluorescence of bacteria without either GFPuv or T7His polymerase was negligible, but the fluorescence caused by other components than GFPuv in the bacteria was still subtracted.

*Results*

The theophylline dependency of the cascade in response to differing concentrations of rhamnose was measured. *E. coli* DH10B-T7His-Theo4 diluted from an overnight culture were grown overnight in a 2 mL culture plate containing a variable amount of L-rhamnose and theophylline. The medium was cleared and the bacteria were resuspended in 50 mM Tris-HCl pH 7.5. The fluorescence was measured at an excitation wavelength of 385 nm and an emission wavelength of 508 nm. The cell density was measured by scattering at 600 nm.

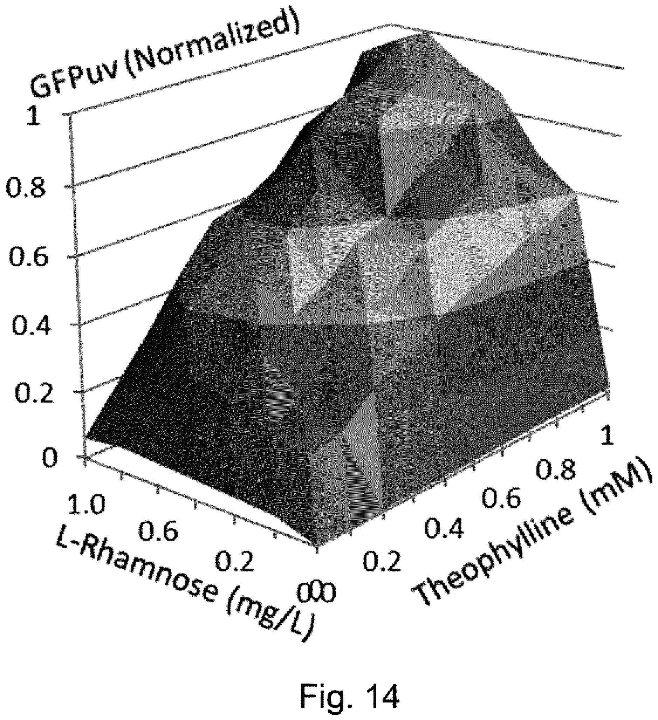

GFPuv fluorescence showed a strong dependency on both L-rhamnose and theophylline (Figure 14). The fluorescence observed without any induction at all does not significantly differ from the fluorescence observed for the GFPuv reporter plasmid alone. This implies that the double control on the T7 polymerase - transcription control by L-rhamnose and translation control by theophylline - succeeds to a large extent in keeping the T7 polymerase inactive. The transcription of T7 polymerase itself dictates the dynamic range in fluorescence caused by theophylline. This dynamic range can be adjusted according to the requirements of the application. At any L-rhamnose concentration, the fold-change caused by theophylline is around 15 times. The cascade being a multi-component system with two types of control makes it very hard to model the dependency on both L-rhamnose and theophylline and to have reproducible results. For a final application as a biosensor, the transcription of T7His polymerase may be fixed with a constitutive promoter, while the functional translation will be ligand dependent.

Since the output dynamic range can be adjusted relatively easily, the intron controlled T7His polymerase can be employed as a generic tool. The intron lowers the maximum translation quite severely, so not all reporter genes will show enough signal when put under control of a ligand dependent intron directly. Enzymes like ThyA or LacZ can handle the lower translation efficiency, but all genes that need an at least decent expression to function, like GFPuv, can now be put under control of one enzyme. An additional advantage is the exchangeability of the reporter plasmids. Expression from these reporter plasmids can be easily adjusted by mutating the T7 promoter.

**Claims**

1. A method of inducing expression of a desired RNA molecule, or a desired protein or polypeptide in a cell comprising:

   (i) providing a cell comprising a first and a second polynucleotide expression construct, the first construct comprising a polynucleotide sequence to be transcribed, and wherein the polynucleotide sequence is interrupted by at least two introns which are self-splicing introns and whose splicing activity is under the control of an aptamer, and wherein the aptamer has binding affinity for an inducer, and the second expression construct comprises a polynucleotide sequence whose expression is under the control of a promoter which is under the control of the RNA, protein or polypeptide product of the first expression construct, and the RNA product of the second expression construct is the desired RNA molecule; or the expressed protein or polypeptide expression product of the second expression construct is the desired protein or polypeptide;
   (ii) exposing the transformed cell to the inducer to activate self-splicing activity of the introns and thereby to produce the desired RNA molecule, or expression of the desired protein or polypeptide.

2. A method of inducing expression as claimed in claim 1, wherein the cell is provided by a step of transforming an host cell with the polynucleotide expression constructs.

3. A method of inducing expression as claimed in claim 1 or claim 2, wherein the cell is transformed with the first and second constructs separately, simultaneously, or sequentially.

4. A method of inducing expression as claimed in any preceding claim, wherein the RNA is a microRNA (miRNA), a small interfering RNA (siRNA), an antisense RNA, a tRNA or a ribozyme.

5. A method of inducing expression as claimed in any preceding claim, wherein the at least two self-splicing introns each comprise an aptamer and wherein the aptamer is the same.

6. A method of inducing expression as claimed in any preceding claim, wherein the self-splicing intron is the T4 *td* gene self-splicing intron.

7. A method of inducing expression as claimed in any preceding claim, wherein the polynucleotide sequence of the first expression construct encodes Phage T7 DNA dependent RNA polymerase.

8. A method of inducing expression as claimed in claim 7, wherein the second expression construct comprises the promoter PT7.

9. A method of inducing expression as claim in any preceding claim, wherein the aptamers bind a ligand; optionally wherein the ligand is selected from one of theophylline, tetracycline, neomycin or malachite green.

10. A cell for inducer molecule-controlled expression of a gene product, the cell comprising first and second polynucleotide expression constructs, wherein the first construct comprises a polynucleotide sequence which is interrupted by at least two introns which are self-splicing introns and whose splicing activity is under the control of an aptamer, and wherein the aptamer has binding affinity for the inducer, and the second expression construct comprises a polynucleotide sequence whose expression is under the control of a promoter whose activity is regulated by the expression product of the first expression construct.

11. A cell as claimed in claim 10, wherein the expressed gene product of the first expression construct is

(a) an RNA; optionally one of a microRNA (miRNA), a small interfering RNA (siRNA), an antisense RNA, a tRNA or a ribozyme; or
(b) a protein or polypeptide.

12. A cell as claimed in claim 10 or claim 11, wherein:

(a) the expressed product of the second expression construct is an RNA molecule; optionally one of a microRNA (miRNA), a small interfering RNA (siRNA), an antisense RNA, a tRNA or a ribozyme; or
(b) the expressed product of the second expression construct is a protein or polypeptide.

13. A cell as claimed in any of claims 10 to 12, wherein the two or more self-splicing introns contain the same aptamer; and/or wherein the or each self-splicing intron is the T4 *td* gene self-splicing intron.

14. A kit for preparing a host cell for inducible host cell expression of a gene product, comprising:

(i) a first polynucleotide expression construct, the first construct comprising a polynucleotide sequence to be transcribed, and wherein the polynucleotide sequence is interrupted by at least two introns which are self-splicing introns and whose splicing activity is under the control of an aptamer, and wherein the aptamer has binding affinity for an inducer;
(ii) a second polynucleotide expression construct, the second construct for receiving a polynucleotide sequence which encodes an RNA or protein or polypeptide to be expressed, and wherein the polynucleotide to be expressed is under the control of a promoter which is inducible by the transcribed polynucleotide.

15. A kit for inducible expression of a gene product, comprising:

(i) a host cell comprising a polynucleotide expression construct, wherein the construct comprises a polynucleotide sequence which is interrupted by at least two introns which are self-splicing introns and whose splicing activity is under the control of an aptamer, and wherein the aptamer has binding affinity for the inducer;
(ii) a second polynucleotide expression construct, the second construct for receiving a polynucleotide sequence which encodes an RNA or protein or polypeptide to be expressed, and wherein the polynucleotide to be expressed is under the control of a promoter which is inducible by the transcribed polynucleotide; optionally
(iii) a set of instructions for (a) inserting a polynucleotide sequence to be expressed into the second polynucleotide and/or (b) transforming the host cell with the second polynucleotide expression construct.

16. A kit as claimed in claim 14 or claim 15, wherein the self-splicing intron is the T4 td gene self-splicing intron; and/or wherein the kit further comprises a container containing an inducer; optionally wherein the inducer is theophylline.

**Patentansprüche**

1. Verfahren zum Induzieren einer Expression eines gewünschten RNA-Moleküls oder eines gewünschten Proteins oder Polypeptids in einer Zelle, wobei das Verfahren folgendes umfasst:

   (i) Bereitstellen einer Zelle, die ein erstes und ein zweites Polynucleotidexpressionskonstrukt umfasst, wobei das erste Konstrukt eine zu transkribierende Polynucleotidsequenz umfasst, und wobei die Polynucleotidsequenz durch mindestens zwei Introns unterbrochen ist, bei denen es sich um selbstspleißende Introns handelt, und wobei deren Spleißaktivität durch ein Aptamer geregelt wird, und wobei das Aptamer eine Bindungsaffinität für einen Induktor aufweist, und wobei das zweite Expressionskonstrukt eine Polynucleotidsequenz umfasst, deren Expression durch einen Promotor geregelt wird, der durch das RNA-, das Protein- oder Polypeptidprodukt des ersten Expressionskonstrukts geregelt wird, und wobei das RNA-Produkt des zweiten Expressionskonstrukts das gewünschte RNA-Molekül ist; oder wobei das exprimierte Protein- oder Polypeptidexpressionsprodukt des zweiten Expressionskonstrukts das gewünschte Protein oder Polypeptid ist;
   (ii) Aussetzen der transformierten Zelle dem Induktor, um die selbstspleißende Aktivität der Introns zu aktivieren und um dadurch das gewünschte RNA-Molekül zu erzeugen oder die Expression des gewünschten Proteins oder Polypeptids.

2. Verfahren zum Induzieren einer Expression nach Anspruch 1, wobei die Zelle bereitgestellt wird durch einen Schritt des Transformierens einer Wirtszelle mit den Polynucleotidexpressionskonstrukten.

3. Verfahren zum Induzieren einer Expression nach Anspruch 1 oder 2, wobei die Zelle mit dem ersten und zweiten Konstrukt separat, gleichzeitig oder sequentiell transformiert wird.

4. Verfahren zum Induzieren einer Expression nach einem der vorstehenden Ansprüche, wobei die RNA eine microRNA (miRNA), eine small interfering RNA (siRNA), eine Antisense RNA, eine tRNA oder ein Ribozym ist.

5. Verfahren zum Induzieren einer Expression nach einem der vorstehenden Ansprüche, wobei die mindestens zwei selbstspleißenden Introns jeweils ein Aptamer umfassen, und wobei das Aptamer jeweils das gleiche ist.

6. Verfahren zum Induzieren einer Expression nach einem der vorstehenden Ansprüche, wobei das selbstspleißende Intron das T4 *td* Gen selbstspleißende Intron ist.

7. Verfahren zum Induzieren einer Expression nach einem der vorstehenden Ansprüche, wobei die Polynucleotidsequenz des ersten Expressionskonstrukts für Phage T7 DNA-abhängige RNA-Polymerase codiert.

8. Verfahren zum Induzieren einer Expression nach Anspruch 7, wobei das zweite Expressionskonstrukt den Promotor PT7 umfasst.

9. Verfahren zum Induzieren einer Expression nach einem der vorstehenden Ansprüche, wobei die Aptamere an einen Liganden binden; wobei der Ligand optional ausgewählt ist aus Theophyllin, Tetracydin, Neomycin und Malachitgrün.

10. Zelle für eine Induktormolekül-geregelt Expression eines Genprodukts, wobei die Zelle erste und zweite Polynucleotidexpressionskonstrukte umfasst, wobei das erste Konstrukt eine Polynucleotidsequenz umfasst, die durch mindestens zwei Introns unterbrochen ist, bei denen es sich um selbstspleißende Introns handelt, und wobei deren Spleißaktivität durch ein Aptamer geregelt wird, und wobei das Aptamer eine Bindungsaffinität für den Induktor aufweist, und wobei das zweite Expressionskonstrukt eine Polynucleotidsequenz umfasst, deren Expression durch einen Promotor geregelt wird, dessen Aktivität durch das Expressionsprodukt des ersten Expressionskonstrukts geregelt wird.

11. Zelle nach Anspruch 10, wobei das exprimierte Genprodukt des ersten Expressionskonstrukts folgendes ist:

    (a) eine RNA; optional eines der folgenden: eine microRNA (miRNA), eine small interfering RNA (siRNA), eine Antisense RNA, eine tRNA oder ein Ribozym; oder
    (b) ein Protein oder ein Polypeptid.

12. Zelle nach Anspruch 10 oder 11, wobei:

(a) das exprimierte Produkt des zweiten Expressionskonstrukts ein RNA-Molekül ist; optional eines der folgenden: eine microRNA (miRNA), eine small interfering RNA (siRNA), eine Antisense RNA, eine tRNA oder ein Ribozym; oder

(b) das exprimierte Produkt des zweiten Expressionskonstrukts ein Protein oder ein Polypeptid ist.

13. Zelle nach einem der Ansprüche 10 bis 12, wobei die zwei oder mehr selbstspleißenden Introns das gleiche Aptamer enthalten; und/oder wobei das oder jedes selbstspleißende Intron das T4 *td* Gen selbstspleißende Intron ist.

14. Kit zur Erzeugung einer Wirtszelle zur induzierbaren Wirtszellenexpression eines Genprodukts, wobei das Kit folgendes umfasst:

(i) ein erstes Polynucleotidexpressionskonstrukt, wobei das erste Konstrukt eine zu transkribierende Polynucleotidsequenz umfasst, und wobei die Polynucleotidsequenz durch mindestens zwei Introns unterbrochen ist, bei denen es sich um selbstspleißende Introns handelt, und wobei deren Spleißaktivität durch ein Aptamer geregelt wird, und wobei das Aptamer eine Bindungsaffinität für einen Induktor aufweist;

(ii) ein zweites Polynucleotidexpressionskonstrukt, wobei das zweite Konstrukt dazu dient, eine Polynucleotidsequenz zu empfangen, die für eine zu exprimierende RNA oder ein Protein oder ein Polypeptid codiert, und wobei das zu exprimierende Polynucleotid durch einen Promotor geregelt wird, der durch das transkribierte Polynucleotid induzierbar ist.

15. Kit für die induzierbare Expression eines Genprodukts, wobei das Kit folgendes umfasst:

(i) eine Wirtszelle, die ein Polynucleotidexpressionskonstrukt umfasst, wobei das Konstrukt eine Polynucleotidsequenz umfasst, die durch mindestens zwei Introns unterbrochen ist, bei denen es sich um selbstspleißende Introns handelt, und wobei deren Spleißaktivität durch ein Aptamer geregelt wird, und wobei das Aptamer eine Bindungsaffinität für einen Induktor aufweist;

(ii) ein zweites Polynucleotidexpressionskonstrukt, wobei das Konstrukt daz dient, eine Polynucleotidsequenz zu empfangen, die für eine zu exprimierende RNA oder ein Protein oder ein Polypeptid codiert, und wobei das Polynucleotid durch einen Promotor geregelt wird, der durch das transkribierte Polynucleotid induzierbar ist; und optional

(iii) eine Reihe von Anweisungen zum (a) Einführen einer zu exprimierenden Polynucleotidsequenz in das zweite Polynucleotid und/oder (b) Transformieren der Wirtszelle mit dem zweiten Polynucleotidexpressionskonstrukt.

16. Kit nach Anspruch 14 oder 15, wobei das selbstspleißende Intron das T4 td Gen selbstspleißende Intron ist; und/oder wobei der Kit ferner ein Behältnis umfasst, das einen Induktor enthält; wobei der Induktor optional Theophyllin ist.

**Revendications**

1. Procédé pour induire l'expression d'une molécule d'ARN souhaitée, ou d'une protéine ou d'un polypeptide souhaité dans une cellule comprenant les étapes consistant à :

(i) fournir une cellule comprenant une première et une seconde construction d'expression de polynucléotide, la première construction comprenant une séquence polynucléotidique à transcrire, et la séquence polynucléotidique étant interrompue par au moins deux introns qui sont des introns à auto-épissage et dont l'activité d'épissage est contrôlée par un aptamère, et l'aptamère ayant une affinité de liaison pour un inducteur, et la seconde construction d'expression comprenant une séquence polynucléotidique dont l'expression est sous le contrôle d'un promoteur qui est sous le contrôle du produit ARN, protéine ou polypeptide selon la première construction d'expression, et le produit ARN de la seconde construction d'expression étant la molécule d'ARN souhaitée ; ou le produit d'expression de la protéine ou du polypeptide exprimé de la seconde construction d'expression étant la protéine ou le polypeptide souhaité ;

(ii) exposer la cellule transformée à l'inducteur pour activer l'activité d'auto-épissage des introns et ainsi produire la molécule d'ARN souhaitée, ou l'expression de la protéine ou du polypeptide souhaité.

2. Procédé d'induction d'expression selon la revendication 1, la cellule étant fournie par une étape de transformation d'une cellule hôte avec les constructions d'expression de polynucléotide.

3.  Procédé d'induction d'expression selon la revendication 1 ou 2, la cellule étant transformée avec les première et seconde constructions séparément, simultanément ou séquentiellement.

4.  Procédé d'induction d'expression selon l'une quelconque des revendications précédentes, l'ARN étant un microARN (miARN), un petit ARN interférent (siRNA), un ARN antisens, un ARNt ou un ribozyme.

5.  Procédé d'induction d'expression selon l'une quelconque des revendications précédentes, les au moins deux introns à auto-épissage comprenant chacun un aptamère et l'aptamère étant le même.

6.  Procédé d'induction d'expression selon l'une quelconque des revendications précédentes, l'intron à auto-épissage étant l'intron à auto-épissage de gène *td* T4.

7.  Procédé d'induction d'expression selon l'une quelconque des revendications précédentes, la séquence polynucléotidique de la première construction d'expression codant pour l'ARN polymérase du Phage T7 dépendant de l'ADN.

8.  Procédé d'induction d'expression selon la revendication 7, la seconde construction d'expression comprenant le promoteur PT7.

9.  Procédé d'induction d'expression selon l'une quelconque des revendications précédentes, les aptamères liant un ligand ; éventuellement le ligand étant choisi parmi la théophylline, la tétracycline, la néomycine ou un vert malachite.

10. Cellule pour l'expression contrôlée par molécule inductrice d'un produit génique, la cellule comprenant des première et seconde constructions d'expression de polynucléotide, la première construction comprenant une séquence polynucléotidique qui est interrompue par au moins deux introns qui sont des introns à auto-épissage et dont l'activité d'épissage est sous le contrôle d'un aptamère, et l'aptamère ayant une affinité de liaison pour l'inducteur, et la seconde construction d'expression comprenant une séquence polynucléotidique dont l'expression est sous le contrôle d'un promoteur dont l'activité est réglée par le produit d'expression de la première construction d'expression.

11. Cellule selon la revendication 10, le produit génique exprimé de la première construction d'expression étant

    (a) un ARN ; éventuellement un parmi un microARN (miARN), un petit ARN interférent (siRNA), un ARN antisens, un ARNt ou un ribozyme ; ou
    (b) une protéine ou un polypeptide.

12. Cellule selon la revendication 10 ou 11,

    (a) le produit exprimé de la seconde construction d'expression étant une molécule d'ARN ; éventuellement un ARN micro (miARN), un petit ARN interférent (siRNA), un ARN antisens, un ARNt ou un ribozyme ; ou
    (b) le produit exprimé de la seconde construction d'expression étant une protéine ou un polypeptide.

13. Cellule selon l'une quelconque des revendications 10 à 12, les deux introns à auto-épissage ou plus contenant le même aptamère ; et/ou le ou chaque intron auto-éplicatif étant l'intron auto-éplicatif du gène *td* T4.

14. Kit pour la préparation d'une cellule hôte pour l'expression inductible d'une cellule hôte d'un produit génique, comprenant :

    (i) une première construction d'expression de polynucléotide, la première construction comprenant une séquence polynucléotidique à transcrire, et la séquence polynucléotidique étant interrompue par au moins deux introns qui sont des introns à auto-épissage et dont l'activité d'épissage est sous le contrôle d'un aptamère, et l'aptamère ayant une affinité de liaison pour un inducteur ;
    (ii) une seconde construction d'expression de polynucléotide, la seconde construction pour recevoir une séquence polynucléotidique qui code pour un ARN ou une protéine ou un polypeptide à exprimer, et le polynucléotide à exprimer étant sous le contrôle d'un promoteur qui est inductible par le polynucléotide transcrit.

15. Kit pour l'expression inductible d'un produit génique, comprenant :

    (i) une cellule hôte comprenant une construction d'expression de polynucléotide, la construction comprenant une séquence polynucléotidique qui est interrompue par au moins deux introns qui sont des introns à auto-épissage et dont l'activité d'épissage est sous le contrôle d'un aptamère, et l'aptamère ayant une affinité de liaison avec l'inducteur ;

(ii) une seconde construction d'expression de polynucléotide, la seconde construction pour recevoir une séquence polynucléotidique qui code pour un ARN ou une protéine ou un polypeptide à exprimer, et le polynucléotide à exprimer étant sous le contrôle d'un promoteur qui est inductible par le polynucléotide transcrit ; éventuellement

(iii) un ensemble d'instructions pour (a) insérer une séquence polynucléotidique à exprimer dans le second polynucléotide et/ou (b) transformer la cellule hôte avec la seconde construction d'expression de polynucléotide.

16. Kit selon la revendication 14 ou 15, l'intron à auto-épissage étant l'intron à auto-épissage du gène td T4 ; et/ou le kit comprenant en outre un récipient contenant un inducteur ; éventuellement l'inducteur étant la théophylline.

Sequence element
complementary to SD region

30S

aptamer
domain

Shine Dalgarno

5'

AUG - 3'

ON

- | + ligand ◇

30S

5'

AUG - 3'

OFF

Shine Dalgarno

## Fig. 1A

Terminator

RNAP

5'

UUUUU — 3'

OFF

- | + ligand ◇

aptamer

ON

5'

UUUUU

RNAP

3'

## Fig. 1B

NO PRODUCT /
METABOLITE

TRANSLATION ✓

C (SHINE - DALGARNO)

PRODUCT / METABOLITE
PRESENT

B

C (SHINE - DALGARNO)

A

TRANSLATION ✗

Fig. 1C

Fig. 2A

Fig. 2B

Fig. 3

Fig. 4

Fig. 5

Fig. 6

ThyA (Exon 1)
GATGTTTTCTTGGGTTAATTGAGGCCTGAGTATAAGGTGACTTATACTTGTAATCTATCTAAACGGGGAA
AspValPheLeuGly

Theophylline Aptamer
GCTCTCTAGTAGACAATCCCGTGCTAAATTGATACCAGCATCGTCTTGATGCCCTTGGCAGCATAAATGC

CTAACGACTATCCCTTTGGGGAGTAGGGTCAAGTGACTCGAAACGATAGACAACTTGCTTTAACAAGTTG

GAGATATAGTCTGCTCTGCATGGTGACATGCAGCTGGATATAATTCCGGGGTAAGATTAACGACCTTATC

ThyA (Exon 2)
TGAACATAATGCTACCGTTTAATATT
LeuProPheAsnIle

## Fig. 7A

pSC026-Theo
3187 bp

P15A Ori (3156-2611)

ThyA Exon 1

T4 Intron

ThyA Exon 2

Kan' (2184-1369)

ThyA (Exon 1)
GATGTTTTCTTGGGTTAATTGAGGCCTGAGTATAAGGTGACTTATACTTGTAATCTATCTAAACGGGGAA
AspValPheLeuGly

Theophylline Aptamer
GCTCTCTAGTAGACAATCCCGTGCTAAATTGATACCAGCATCGTCTTGATGCCCTTGGCAGCATAAATGC

CTAACGACTATCCCTTTGGGGAGTAGGGTCAAGTGACTCGAAACGATAGACAACTTGCTTTAACAAGTTG

GAGATATAGTCTGCTCTGCATGGTGACATGCAGCTGGATATAATTCCGGGGTAAGATTAACGACCTTATC

ThyA (Exon 2)
TGAACATAATGCTACCGTTTAATATT
LeuProPheAsnIle

# Fig. 7B

AAATATTTCTAGATTTCAGTGCAATTTATCTCTTCAAATGTAGCACCTGAAGTCAGCCCCATACGATATA

AGTTGTAATTCCATATGTCATGTTTGACAGCTTATCATCGATAAGCTTTAATGCGGTAGTTTATCACGCA

ATGAGCTTGCACTGCAGAACTTTCTTAAGGAGATAAAGCAATGAAATCTAACAATGCGCTCATCGTCATC
                                            MetLysSerAsnAsnAlaLeuIleValIle

# Fig. 7C

Fig. 8

Fig. 9

Fig. 10A

DNA

T4 Intron · · · · · · · · · · · · · · · · · · · · · T4 Intron

Exon 1 · · · · Exon 2 · · · · Exon 3

Pre mRNA

Cofactor binding domain · · · · · · · · · Cofactor binding domain

Exon 1 · · · · Exon 2 · · · · Exon 3

Cofactor

Mature mRNA

Exon 1 · Exon 2 · Exon 3 · mRFP

Complete splicing of Exon 2 introduces a frameshift
Presence of any intron introduces multiple stop codons

Protein

ThyA

Background factors
ThyA: Uninduced splicing of the introns

## Fig. 10B

EP 3 283 628 B1

Fig. 10C

Fig. 10D

## T7 Polymerase on Genome DNA

Rhamnose inducible promoter

Exon 1    Exon 2

Inducer compound
added to the medium

T7 RNA polymerase mRNA

Exon 1    Exon 2    Exon 3

Maturation

Mature T7 RNA polymerase mRNA

Exon 1    Exon 2    Exon 3    mRNA

Translation

T7 RNA polymerase Protein

# Fig. 11

Fig. 12

Fig. 13

Fig. 14

Fig. 15

## REFERENCES CITED IN THE DESCRIPTION

**Patent documents cited in the description**

- WO 2008116220 A2

- WO 2012153142 A2

**Non-patent literature cited in the description**

- **GROHER F. ; SUESS B.** Synthetic riboswitches - A tool comes of age. *Biochimica et Biophysica Acta,* 2014, vol. 1839, 964-973
- **SUESS et al.** *Nucleic Acids Res,* 2004, vol. 32, 1610-1614
- **OGAWA ; MAEDA.** *Bioorg Med Chem Lett,* 2007, vol. 17, 3156-3160
- **TOPP ; GALLIVAN.** *RNA,* 2008, vol. 14, 2498-2503
- **MANDAL ; BREAKER.** *Nat Rev Mol Cell Biol,* 2004, vol. 5, 451-463
- **PARASKEVA et al.** *PNAS,* 1998, vol. 95, 951-956
- **STRIPECKE et al.** *Mol. Cell. Biol.,* 1994, vol. 14, 5898-5909
- **DE SMIT ; VAN DUIN.** *PNAS,* 1990, vol. 87, 7668-7672
- **WERSTUCK ; GREEN.** *Science,* 1998, vol. 282, 296-298
- **DESAI ; GALLIVAN.** *J. Am. Chem. Soc.,* 2004, vol. 126, 13247-13254

- **SUESS et al.** *Nucleic Acids Res.,* 2004, vol. 32, 1610-1614
- **THOMPSON et al.** *BMC Biotechnol.,* 2002, vol. 2, 21
- **SUESS et al.** *Nucleic Acids Res.,* 2003, vol. 31, 1853-1858
- **OGAWA ; MAEDA.** *Bioorg. Med. Chem. Lett.,* 2007, vol. 17, 3156-3160
- **MANDAL ; BREAKER.** *Nat Rev Mol Cell Biol,* 2004, vol. 5, 451-463
- **CHEAH M. T. et al.** Control of alternative RNA splicing and gene expression by eukaryotic riboswitches. *Nature,* 2007, vol. 447 (7143), 497-500
- **PICHLER ; SCHROEDER.** *J Biol Chem,* 2002, vol. 277, 17987-17993
- **DATSENKO ; WANNER.** *PNAS,* 2000, vol. 97, 6640-6645
- **THOMPSON et al.** *BMC Biotechnol,* 2002, vol. 2, 21